# EUROPEAN PATENT SPECIFICATION

(11) **EP 4 262 663 B1**
(45) Date of publication and mention of the grant of the patent: **18.06.2025**
(21) Application number: 21843692.1
(22) Date of filing: 20.12.2021
(51) Int. Cl.: A61F 13/475, A61F 13/532, A61F 13/537

(54) **ABSORBENT ARTICLE WITH IMPROVED STRUCTURE**
ABSORBIERENDER ARTIKEL MIT VERBESSERTER STRUKTUR
ARTICLE ABSORBANT À STRUCTURE AMÉLIORÉE

(30) Priority: 18.12.2020 NL 2027163; 12.05.2021 NL 2028199; 23.07.2021 EP 21187395
(43) Date of publication of application: 25.10.2023
(60) Divisional application: 25164310.2
(73) Proprietor: Drylock Technologies NV, 9240 Zele (BE)
(72) Inventor: DERYCKE, Tom, 9240 Zele (BE); SMET, Steven, 9240 Zele (BE); VAN MALDEREN, Bart, 9240 Zele (BE)
(74) Representative: Arnold & Siedsma
(86) International application number: PCT/EP2021/086890
(87) International publication number: WO 2022/129642

(56) References cited:
- EP-A1- 3 388 039
- EP-A1- 3 560 469
- EP-A1- 3 861 970
- EP-B1- 3 403 630
- WO-A1-00/59430
- WO-A1-2015/031229
- WO-A1-2017/095399
- WO-A2-02/067809

## Description

### TECHNICAL FIELD

The present invention pertains to the technical field of absorbent articles, more preferably disposable personal care articles such as diapers, baby pants, adult incontinent garments, and the like, and to absorbent structures for use in such absorbent articles. More specifically the present invention relates to an absorbent article comprising an absorbent core with one or more channels between a topsheet and a backsheet. The present invention also relates to methods for manufacturing such an absorbent article.

### BACKGROUND

Absorbent articles comprising an absorbent core with one or more channels between a topsheet and a backsheet are well known. Examples of such absorbent articles are described for example in patents EP 3 403 630 B1, EP 3 403 632 B1 and EP 3 403 631 A1 in the name of the applicant.

It is known to include a liquid-impermeable, either hydrophilic or hydrophobic, distribution or wicking layer which helps to wick and transport liquids and having a capability to disperse the liquid over the surface of said wicking layer from the less absorbent area's (e.g. saturated) to the more absorbent area's (e.g. unsaturated). The distribution layer or wicking layer is preferably located at the garment facing side of the absorbent core. Due to the specific absorbent capacity of the distribution or wicking layer, the liquid in the one or more channels will be drawn up into the distribution layer and will spread out throughout the rest of the distribution layer. In that way, the distribution, transport and absorption of the liquid can be improved.

It is further known to provide a liquid distribution and acquisition layer (ADL) between the topsheet and the absorbent core in order to take up and distribute the liquid from the topsheet towards the absorbent core. Typically, an ADL extends over an area where liquid insult is to be expected. Known ADL layers have the disadvantage that it takes some time before a dry feeling is obtained at the topsheet surface and that rewetting can be significant.

WO 2015/031229 A1 is directed, in part, to an absorbent article that comprises a liquid management system (LMS) and an absorbent core disposed at least partially intermediate a topsheet and a backsheet. The LMS defines one or more channels substantially free of superabsorbent polymers. The one or more channels of the LMS may at least partially overlap channels defined by the absorbent core. The absorbent article may comprise a liquid distribution system (LDS) that defines one or more channels.

EP 3 388 039 A1 discloses a polymer provided region (13) at which a superabsorbent polymer (12) is provided, and a polymer non-provided region (14) at which the superabsorbent polymer (12) is not provided are formed, a liquid diffusion sheet (15), that promotes diffusion of body fluid in a plane direction, is staked to be adjacent to a skin side surface of a lower layer sheet (11), and an upper layer sheet (10) is bonded with the liquid diffusion sheet (15) under a state that the upper layer sheet (10) contacts the liquid diffusion sheet (15), at the polymer non-provided region (14) to form a polymer sheet (4).

US 2017/0079858 A1 discloses an absorbent article including a channelled absorbent core and an air-through bonded carded nonwoven layer as a topsheet or an acquisition layer under the topsheet.

### SUMMARY

The object of embodiments of the invention is to provide an absorbent article of the type stated in the preamble, with an improved balance between the distribution via the capillary acceleration sheet and the top core wrap, on the one hand, and the bottom core wrap, on the other hand, whilst maintaining a good integrity of the absorbent article, and such that a dry feeling is obtained in a fast manner after a liquid insult.

According to a first aspect of the invention, there is provided an absorbent article comprising a liquid pervious topsheet, a liquid impervious backsheet, an absorbent core positioned between the liquid pervious topsheet and the liquid impervious backsheet, said absorbent core comprising a bottom core wrap, a top core wrap and absorbent material between the bottom core wrap and the top core wrap, and a capillary acceleration sheet between the absorbent core and the liquid pervious topsheet. The absorbent material comprises superabsorbent particles and optionally cellulosic fluff pulp, wherein the absorbent material is arranged such that one or more channels are formed, wherein less absorbent material per surface area is present in the one or more channels compared to an area around the one or more channels, wherein preferably substantially no absorbent material is present in the one or more channels. The capillary acceleration sheet is a through-air-bonded nonwoven having a basis weight between 20 and 30 gsm.

By using such relatively light but dense through-air-bonded nonwoven a good topsheet dryness, and in particular a fast topsheet dryness after liquid insult, and a good rewetting behaviour, is obtained. By combining at least one channel with a capillary acceleration sheet a fast dryness feeling and a low rewetting at the level of the topsheet can be ensured, whilst at the same time providing a good liquid distribution through the one or more channels resulting in improved leakage protection.

According to a preferred embodiment, the bottom core wrap has a basis weight which is larger than the basis weight of the top core wrap and/or larger than the basis weight of the capillary acceleration sheet.

By providing a bottom core wrap having a basis weight which is larger than the basis weight of the top core wrap and/or larger than the basis weight of the capillary acceleration sheet, the bottom core wrap can be used for distribution purposes, distributing the liquid from the one or more channels to the absorbent material, whilst the providing integrity to the absorbent article. The liquid may be transported from the one or more channels through the bottom core wrap, along areas where the absorbent material is saturated, e.g. areas close to the one or more channels, to other areas where the absorbent material is not yet saturated. Further, by using a relatively light top core wrap and/or capillary acceleration sheet it takes less time before a dry feeling is obtained at the topsheet surface and any rewetting is reduced or avoided.

Preferred embodiments are disclosed in the dependent claims.

Preferably, the capillary acceleration sheet has been subjected to a hydrophilic spin finish treatment. This further improves the topsheet dryness.

Preferably, each channel has a width measured in a transverse direction of the absorbent core, said width being at least 5% of the width of the absorbent core, preferably at least 7%, e.g. at least 10%, e.g. at least 15% or even at least 20%. Preferably, the width of each channel is less than 30% of the width of the absorbent core, e.g. less than 25%. In this way, the risk of having channel portions where fluff is present, is reduced, allowing an improved attachment in the one or more channels.

The width of the absorbent core is measured in a transverse direction of the absorbent core, in an area where the respective one or more channels are present, between two end points where absorbent material is present, i.e. it is the width of the absorbent material deposition area of the absorbent core. Thus, the top core wrap sheet and/or the back core wrap sheet may extend over a width which is larger than the width of the absorbent core, as they may be attached along a periphery where no absorbent material is present. In the area where the one or more channels are present, the width of the absorbent core may vary between a minimum width and a maximum width, and preferably the above specified minimum width of each channel is valid both for the minimum and the maximum width. The absorbent material deposition area may thus have a width which varies e.g. between a value above 100 mm and a value below 100 mm. Preferably the difference is at least 5 mm. For example, a width of the absorbent material deposition area measured at a front end of the absorbent core may be above 100 mm, or even above 105 mm and a width measured at a rear end of the absorbent core may be below 100 mm, or even below 95 mm. The width of the channel may be constant through substantially the whole length of the channel or may vary along its length. When the width varies along the length, then the above specified width percentages apply for the average width of the respective channel. Preferably, the one or more channels are formed by one or more absorbent material-free zones within the absorbent material deposition area of the absorbent core, and the width of a channel is considered to be the width of the material-free zone, disregarding the possible presence of core wrap within the channel. If the one or more channels are formed by one or more absorbent material zones where less absorbent material per surface area is present as compared to an area around the one or more channel, the width of the channel is the width of the absorbent material zone where less absorbent material per surface area is present.

The absorbent core periphery, which may be the periphery of the top/bottom core wrap, may define any suitable shape, such as a "T," "Y," "hour-glass," or "dog-bone" shape, for example. A "T shape may have a larger width in the front portion than in the rear portion of the absorbent core. An absorbent core periphery having a generally "dog bone" or "hour-glass" shape may taper along its width towards the middle or "crotch" region of the core. In this way, the absorbent core may have a relatively narrow width in an area of the absorbent core intended to be placed in the crotch region of an absorbent article.

Preferably, the bottom core wrap consists of a single sheet. In that way the bottom core wrap can be easily transported in a machine for producing the absorbent articles. Further, the strength and/or integrity of the absorbent article may be increased.

Preferably, the bottom core wrap comprises a through-air bonded non-woven, wherein preferably the bottom core wrap consists of a through-air bonded non-woven. Preferably, the bottom core wrap has a basis weight between 18 and 80 g/m2, preferably between 20 and 75 g/m2, more preferably between 25 and 70 g/m2, even more preferably between 30 and 65 g/m2.

The capillary acceleration sheet is a through-air bonded non-woven and has a basis weight between 20 and 30 g/m2.

Preferably, the top core wrap comprises at least one spunbond and optionally at least one meltblown layer, wherein more preferably the top core wrap consists of a spunbond layer. Preferably, the top core wrap has a basis weight between 8 and 30 g/m2.

Preferably, an amount of cellulosic fluff pulp in the absorbent material is below 250 g/m2, preferably below 200 g/m2, more preferably below 180 g/m2, even more preferably below 160 g/m2, e.g. between 60 g/m2 and 180 g/m2 or between 70 g/m2 and 170 g/m2.

Preferably, an amount of superabsorbent particles in the absorbent material is above 300 g/m2, preferably above 320 g/m2, more preferably above 340 g/m2, e.g. between 300 g/m2 and 400 g/m2 or between 320 g/m2 and 390 g/m2.

Preferably, the superabsorbent particles have a permeability SFC which is higher than 10 x 10⁻⁷ cm³.S.g ¹ and/or a permeability GBP which is higher than 20 Da, preferably higher than 25 Da.

Preferably, the absorbent material comprises more than 60 weight%, preferably more than 70 weight% superabsorbent particles.

In an exemplary embodiment, the bottom core wrap is a through-air bonded non-woven. This may be a spunbond/spunbond through-air-bonded nonwoven, or a classical through-air bonded nonwoven obtained by sequentially feeding the fibres of the first and second layer.

In an exemplary embodiment, the bottom core wrap comprises a first layer and a second layer, wherein said first layer has a first density between 20 and 150 kg/m3 and said second layer has a second density between 100 and 400 kg/m3, said second density being higher than the first density, wherein said first layer is closer to the absorbent material than said second layer. Preferably, a difference between the first and the second density is higher than 20 kg/m3, more preferably higher than 30 kg/m3 even more preferably higher than 40 kg/m2, even more preferably between 50 and 150 kg/m3, most preferably between 60 and 140 kg/m3, e.g. between 70 and 130 kg/m3. By using such differences in density a good balance between distribution and integrity properties can be achieved.

In an exemplary embodiment, the first layer is a through-air-bonded carded non-woven and the second layer is a spunbond layer, e.g. a PP spunbond layer.

In another exemplary embodiment, the first layer is a spunbond layer and the second layer is a spunbond layer. Thus, the first layer may be a more porous spunbond layer than the second layer.

The first and second layer may be obtained using multiple spinning column, whereupon the assembly is interconnected using e.g. through-air-bonding.

In a preferred embodiment, the first layer has a first basis weight and the second layer has a second basis weight, said second basis weight being lower than said first basis weight. Thus, the second layer may be a rather thin layer as it does not have to contribute to the distribution. Preferably, the difference between the first layer and the second layer is larger than 1 g/m2, more preferably larger than 2 g/m2, even more preferably larger than 3 g/m2, or even larger than 5 g/m2, for example between 1 and 10 g/m2.

In an exemplary embodiment, the first layer has a first basis weight between 15 and 80 g/m2, preferably between 25 and 80 g/m2, and/or the second layer has a second basis weight between 8 and 35 g/m2, preferably between 8 and 25 g/m2.

Preferably, the first layer and/or the second layer comprise more than 90weight% of synthetic fibres, preferably more than 95weight% of synthetic fibres.

In a preferred embodiment, the first layer comprises polyester fibres. Polyester fibres have the advantage of being capable of providing a highly voluminous first layer, resulting in good distribution properties.

In a possible embodiment, the second layer comprises any one of the following fibres or a combination thereof: polyethylene, polyester, copolyester, polypropylene, polyactic acid (PLA).

Preferably, the second density is selected such that it forms a barrier for the superabsorbent particles. For example, the second layer may have a mean flow pore size below a predetermined value, and the absorbent material comprises superabsorbent particles prepared such that substantially no superabsorbent particles having dimensions smaller than said mean flow pore size are present in the absorbent material. By using absorbent material comprising absorbent particles with a well-defined particle size distribution (PDS) adapted to the flow pore size of the bottom core wrap, and in particular the second layer thereof, any escaping of absorbent material can be reduced. Topping of the fines (e.g. a "dust-fraction" < 50 µm) away may be advantageous, as well as the removal of large fraction (e.g. >600 µm). For example, traditional blend polymerization superabsorbent particles can serve for this purpose, with sieve selection. According to another example drop polymerization superabsorbent particles (e.g. SAVIVA^{®} superabsorbent of the company BASF) with a narrow PDS can be used. Also solvent polymerized superabsorbent particles can have such a narrow PDS.

In an exemplary embodiment, the mean flow pore size of the bottom core wrap is below 200 micron, more preferably below 150 micron, even more preferably between 10 and 150 micron, and most preferably between 20 and 150 micron. These values may be achieved thanks to the presence of the second layer.

Preferably, the bottom core wrap is attached to the top core wrap and/or the capillary acceleration at least in a portion of the one or more channels. In that manner, a well structured absorbent article is obtained with one or more well defined channels, further improving the liquid guidance by mass flow through the one or more channels, through the bottom core wrap to the absorbent material.

Preferably, the bottom distribution sheet is attached along a periphery to the bottom core wrap and/or the capillary acceleration sheet.

In an exemplary embodiment, the bottom core wrap is glued to the top core wrap and/or a capillary acceleration at least in a portion of the one or more channels. For example, adhesive may be arranged in one or more lanes or in a swirl pattern on the top core wrap and/or the capillary acceleration sheet and/or the bottom core wrap, such that the one or more lanes or swirl pattern overlap at least partially with the one or more channels. Since the first layer is a relatively porous layer it will allow glue to pass through the first layer to be adhered to the second layer.

In addition or alternatively, the bottom core wrap is sealed in accordance with a sealing pattern to the top core wrap and/or to the capillary acceleration at least in a portion of the one or more channels, wherein preferably the sealing is realized by heat and/or pressure and/or ultrasonic energy. For example, the sealing pattern covers less than 80%, preferably less than 70%, more preferably less than 60%, even more preferably less than 50%, most preferably between 1 and 50% of the surface area of the one or more channels. For example, the sealing pattern covers between 1 and 50% of the total surface area of the one or more channels, or between 1 and 40%, or between 1 and 30%, or between 1 and 25%, or between 2 and 25%, or between 3 and 25%, or between 4 and 25%.

Such sealing pattern can provide a good resistance against the swell forces generated by liquid being captured in the superabsorbent particles by hydrogen bonds, but the sealing pattern equally can provide resistance against dry and wet superabsorbent particles trying to penetrate through the second layer and through the sealing pattern after as well as during the sealing step of the manufacturing process. In that manner, any risk of dry or wet SAP particles coming into contact with sensitive skin upon wearing is avoided or reduced. Further, by having a reduced surface area that is being sealed in accordance with a pattern, any particles remaining in the one or more channel zones can easily migrate to a non-sealing area so that the risk of creating holes in the one or more channel zones is reduced or avoided.

Preferably, the sealing pattern is a regular pattern, such as a line pattern such as a grid, a pattern of dots, etc. The dots may have any shape, e.g. round, polygonal, etc. The line pattern may comprise one or more sets of parallel lines. When a first set of parallel lines and a second set of parallel lines is included, the lines of the first set may be oriented at a non-zero angle with respect to the lines of the second set.

Preferably, the sealing pattern comprises a large number of distinct sealing areas spread across the one or more channels. Preferably, the large number is larger than 10, more preferably larger than 20.

Preferably, the sealing pattern comprises a plurality of discrete elements, and each discrete element has a first dimension in a first direction and a second dimensions in a direction perpendicular to the first direction. The first dimension is smaller than 2 mm, preferably smaller than 1.5 mm, more preferably smaller than 1 mm, e.g. between 0.1 and 0.7 mm or between 0.2 and 0.7 mm, or between 0.3 and 0.6 mm.

By providing well selected sealing patterns, such as grids, dots, polygons, etc., that have a limited total bonding area with fine single bonding areas, e.g. having a surface area smaller than 2 mm² or a line thickness lower than 1 mm, individual superabsorbent particles will find easier their way to non-sealed zones in order not to create any hole.

For determining the density of the bottom core wrap, the mass per unit area may be measured using NWSP 130.1.R0 (20) and the nonwoven thickness may be measured using NWSP 120.6.R0 (15). The density can then be calculated as the mass per unit area divided by the thickness.

In an exemplary embodiment, the capillary acceleration sheet has a mean flow pore size below a predetermined value, and the absorbent material comprises superabsorbent particles prepared such that substantially no superabsorbent particles having dimensions smaller than said mean flow pore size are present in the absorbent material. By using absorbent material comprising absorbent particles with a well-defined particle size distribution (PDS) adapted to the flow pore size of the capillary acceleration sheet, any escaping of absorbent material through the topsheet can be further reduced. Topping of the fines (e.g. a "dust-fraction" < 50 µm) away may be advantageous, as well as the removal of large fraction (e.g. >600 µm). For example, traditional blend polymerization superabsorbent particles can serve for this purpose, with sieve selection. According to another example drop polymerization superabsorbent particles (e.g. SAVIVA^{®} superabsorbent of the company BASF) with a narrow PDS can be used. Also solvent polymerized superabsorbent particles can have such a narrow PDS.

The capillary acceleration sheet may have a mean flow pore size below 50 micron, preferably between 5 and 50 micron, more preferably between 10 and 50 micron, e.g. between 10 and 40 micron or between 20 and 40 micron.

By having a capillary acceleration sheet with a mean flow pore size below 50 micron, the absorbent material, and in particular super absorbent particles can be well maintained in the absorbent article without requiring an adapted topsheet. On the other hand, the capillary acceleration sheet can fulfil its capillary acceleration function, and will allow liquid to pass through the capillary acceleration sheet. By combining at least one channel with a capillary acceleration sheet a fast dryness feeling and a low rewetting at the level of the topsheet can be ensured, whilst at the same time providing a good liquid distribution through the one or more channels resulting in improved leakage protection. Also, the topsheet may be an apertured topsheet as the low mean flow pore size of the capillary acceleration sheet will avoid that any significant amounts of absorbent material, and in particular of superabsorbent particles, escape through the topsheet.

The measure "mean flow pore size" refers to a measure of average pore diameter as determined by a liquid displacement technique utilizing a Coulter Porometer and Coulter POROFIL^{®} test liquid available from Coulter Electronics Limited, Luton, England. The mean flow pore size is determined by wetting a test sample with a liquid having a very low surface tension (i.e., Coulter POROFIL^{®}). Air pressure is applied to one side of the sample. Eventually, as the air pressure is increased, the capillary attraction of the fluid in the largest pores is overcome, forcing the liquid out and allowing air to pass through the sample. With further increases in the air pressure, progressively smaller and smaller holes will clear. A flow versus pressure relationship for the wet sample can be established and compared to the results for the dry sample. The mean flow pore size is measured at the point where the curve representing 50% of the dry sample flow versus pressure intersects the curve representing wet sample flow versus pressure. The diameter of the pore which opens at that particular pressure (i.e., the mean flow pore size) can be determined from the following expression: pore diameter µm = 40 τ / pressure, where τ = surface tension of the fluid expressed in units of mN/M; the pressure is the applied pressure expressed in millibars (mbar); and the very low surface tension of the liquid used to wet the sample allows one to assume that the contact angle of the liquid on the sample is about zero.

The capillary acceleration sheet may have a mean flow pore size below a predetermined value, and the absorbent material comprises superabsorbent particles prepared such that substantially no superabsorbent particles having dimensions smaller than said mean flow pore size are present in the absorbent material.

By using a capillary acceleration sheet having a mean flow pore size below a predetermined value in combination with absorbent particles with a well-defined particle size distribution (PDS) adapted to the predetermined value, any escaping of absorbent material through the topsheet can be avoided or reduced. For example, traditional blend polymerization superabsorbent particles can serve for this purpose, wherein a sieve selection is used to obtain the desired PDS. According to another example drop polymerization superabsorbent particles (e.g. SAVIVA^{®} superabsorbent of the company BASF) with a narrow PDS can be used. Also solvent polymerized superabsorbent particles can have such a narrow PDS.

Preferably, the mean flow pore size is between 10 and 200 micron, more preferably between 10 and 150 micron, even more preferably between 20 and 150 micron, and most preferably between 30 and 150 micron. For example, the mean flow pore size is between 30 and 120 micron or between 30 and 100 micron.

In an exemplary embodiment, the capillary acceleration sheet is sealed to the top core wrap according to the sealing pattern, and optionally also to the bottom core wrap through the top core wrap.

Preferably, the sealing is realized by heat and/or pressure and/or ultrasonic energy. Preferably, the sealing is done by pressing one or more sealing bars against one or more zones overlapping with the one or more channels, said sealing bars being provided with protrusions according to the sealing pattern.

Preferably, the capillary acceleration sheet is a nonwoven comprising fibres having an average diameter between 10 and 35 micron, more preferably between 15 and 30 micron, even more preferably between 17 and 27 micron.

More in particular, the capillary acceleration sheet may have a fineness which results in a high capillary suction of liquid towards the absorbent material, where the liquid is further distributed through the at least one channel and absorbed by the absorbent material. Thus, the capillary acceleration sheet pulls liquid to the absorbent material in a very fast manner, resulting in a fast dryness at top sheet level.

In an exemplary embodiment, the capillary acceleration sheet has an average fineness which is between 2-6 dtex, i.e. between 1.8 and 5.4 den. It is noted that instead of or in addition to defining the average diameter of the capillary acceleration sheet, this sheet may also be defined using a fineness value, such as a dtex value or a den value. A den value may be determined using test method ASTM 1577.

Preferably, the capillary acceleration sheet is attached to the bottom core distribution assembly at least in a portion of the one or more channels. The attachment may be a continuous or discontinuous attachment. For example, the capillary acceleration sheet may be attached to the bottom core wrap along a major portion of the one or more channels, with one or more unattached portions being present between attached portions. The one or more channels may comprise one or more permanent attachment portions which remain attached after wetting and/or one or more semi-permanent attachment portions which detach after wetting and/or one or more unattached portions. An unattached portion or a semi-permanent attachment portion allows for an increased swelling of the absorbent material after the one or more channels have fulfilled their function of distributing the liquid, as wet absorbent material is allowed to extend/swell into the channel area in case of an unattached or detached portion.

According to an exemplary embodiment, the capillary acceleration sheet is a nonwoven with polypropylene (PP) fibres. The advantage of PP is that it can be easily shaped.

According to an exemplary embodiment, the topsheet is an apertured topsheet having apertures with a surface area larger than 0.1 mm2, preferably larger than 0.5 mm2. This allows liquid to pass easily through the topsheet towards the capillary acceleration sheet. Especially for embodiments where the capillary acceleration sheet forms a barrier for superabsorbent particles such an embodiment is advantageous.

According to an exemplary embodiment, the capillary acceleration sheet comprises a blend of fibres having different diameters. Preferably, the fibres are intermingled, i.e. preferably the different fibres are not arranged in layers but mixed with each other to form the capillary acceleration sheet. Preferably, a blend of polypropylene (PP) fibres is used. However, it is also possible to use other single component or bi-component fibres, such as polyethylene (PE) fibres, polyethylene-terephthalate (PET) fibres, PE/PP bi-component fibres, PE/PLA bi-component fibres, etc. The fibres may be continuously crimped fibres.

According to an exemplary embodiment, the capillary acceleration sheet comprises a blend of first fibres having an average first diameter above 20 micron, preferably above 22 micron, more preferably above 24 micron, and second fibres having an average second diameter below 20 micron, preferably below 19 micron, more preferably below 18 micron. In that manner, the fine second fibres will contribute to the fast capillary suction whilst the coarser first fibres will ensure a good distribution of the liquid, also by mass flow.

Preferably, the first fibres represent less than 50 weight% of the capillary acceleration sheet, more preferably less than 45 weight%, and the second fibres represent more than 50 weight% of the capillary acceleration sheet, more preferably more than 55 weight%. Such a distribution provides a good balance between suction and migration by capillary flow promoted by the finer second fibres and liquid distribution by mass flow promoted by the coarser first fibres.

According to an exemplary embodiment, the capillary acceleration sheet comprises a fraction of fibres having an average diameter which is at least 10% lower than an average diameter of the fibres of the liquid pervious topsheet, preferably at least 20% lower than an average diameter of the fibres of the liquid pervious topsheet. By having finer fibres in the capillary acceleration sheet than in the topsheet, the liquid will pass fast through the topsheet and the main capillary suction will take place in the capillary acceleration sheet.

Preferably, the topsheet has a basis weight between 10 and 30 g/m², preferably between 15 and 25 g/m². Preferably, the fibres of the topsheet have an average fineness between 1-3 dtex. Preferably, the topsheet is any one of the following: a spunbond nonwoven, a through air bonded carded web (TABCW). Preferably, the through air bonded carded web comprises polyethylene/polypropylene bi-component staple fibres.

According to an exemplary embodiment, at least a portion of the topsheet is bonded to the capillary acceleration sheet, preferably at least in a portion of the one or more channels.

According to an exemplary embodiment, the capillary acceleration sheet extends over at least 15%, preferably at least 20%, more preferably at least 45% of an upper surface area of the bottom core wrap; and/or the capillary acceleration sheet extends over at least 25%, preferably at least 30%, more preferably at least 35% of a length of the bottom core wrap; and/or wherein the capillary acceleration sheet extends over at least 25%, preferably at least 30%, more preferably at least 35% of a width of the bottom core wrap.

According to an exemplary embodiment, the bottom core wrap has a first and second longitudinal edge and a first and second transverse edge and the one or more channels comprise at least one elongate channel extending from a crotch region in the direction of the first and/or second transverse edge. Such channels will allow a good liquid distribution by mass flow between the first and second transverse edge.

According to an exemplary embodiment, the one or more channels comprise one or more attachment zones where the capillary acceleration sheet and the top core wrap are attached to the back core wrap sheet. Preferably, the one or more attachment zones are formed by any one of the following or a combination thereof: pressure bonding, thermal bonding, sonic bonding, chemical bonding, adhesive. According to an exemplary embodiment, the one or more channels comprise any one or more of the following: one or more permanent attachment zones, one or more semi-permanent attachment zones configured to release after having been in contact with liquid, for example after having been in contact with the liquid for a time period of less than 2 minutes, one or more unattached zones.

According to an exemplary embodiment, a width of the one or more channels, seen in a transverse direction of the bottom core wrap, is at least 4 mm, preferably at least 6 mm, more preferably at least 8 mm, most preferably at least 9 mm, e.g. between 10 mm and 20 mm or between 12 mm and 20 mm. In that manner, it can be ensured that a sufficient amount of liquid can be distributed and any leakage can be limited or avoided. Further, by having wider channels, the risk of getting fluff material in the one or more channels may be reduced. The width of the channel may be constant through substantially the whole length of the channel or may vary along its length. When the width varies along the length, then the above specified width values apply for the average width of the respective channel.

According to an exemplary embodiment, the one or more channels comprise a first channel and a second channel which extend next to each other and are each extending in the direction of a first and/or second transverse edge of the bottom core wrap. Preferably, the first and second channel are oriented substantially in a longitudinal direction of the bottom core wrap. Preferably, the capillary acceleration sheet at least partially covers the first and second channel. In that manner, the capillary acceleration sheet can suck liquid towards the two channels.

According to an exemplary embodiment, the distance between the first and the second channel is between 10 mm and 50 mm, e.g. between 15 mm and 30 mm; and/or the length of the first and the second channel is larger than 60 mm, preferably larger than 70 mm. Such dimensions will typically result in a good distribution. The skilled person understands that the dimensions will be dependent on the size of the absorbent article, and thus one the size of the bottom core wrap.

According to an exemplary embodiment, the one or more channels further comprise a third and a fourth channel located at a distance of each other and at a distance of the first and second channel, said third and fourth channel each extending in the direction of the first and/or second transverse edge. Preferably, the capillary acceleration sheet at least partially covers the third and fourth channel. By adding such a third and fourth channel the distribution can be further enhanced.

Preferably, the distance between the first and the second channel is different from the distance between the third and the fourth channel. Preferably the distance between the first and the second channel is between 20 mm and 50 mm, and the distance between the third and the fourth channel is between 10 mm and 30 mm.

Preferably, the bottom core wrap has a front portion extending at one side of a transverse crotch line and a rear portion extending at the other side of the transverse crotch line; wherein the first and second channel extend at least in the front portion of the bottom core wrap; and wherein the third and fourth channel extend at least in the rear portion of the bottom core wrap. Preferably, the distance between the first and the second channel is smaller than the distance between the third and the fourth channel.

Optionally, the first channel may be connected to the third channel through a first semi-permanent attachment zone and the second channel may be connected to the fourth attachment zone through a second semi-permanent attachment zone, wherein the first, second, third and fourth channels are formed by permanent attachment zones.

According to an exemplary embodiment, the one or more channels comprise at least a first and a second attachment zone located a distance of each other, said first and second attachment zone each extending from a crotch region in the direction of a first and/or second transverse edge of the bottom core wrap.

According to an exemplary embodiment, a position and/or shape of the one or more channels is indicated by means of a distinguishable colour and/or coloured pattern, e.g. by a printed ink layer. For example, the distinguishable colour and/or coloured pattern may be provided on at least one of the topsheet, the capillary acceleration sheet, the backsheet and the bottom core wrap.

According to an exemplary embodiment, the one or more channels together extend over a channel length which is at least 20 %, preferably at least 30%, preferably at least 40%, more preferably at least 50% of a length of the bottom core wrap.

According to an exemplary embodiment, the one or more channels and the capillary acceleration sheet are arranged symmetrically with respect to a longitudinal centre line of the bottom core wrap. Preferably, the absorbent material comprises cellulosic fluff pulp made up of cellulose fibres and superabsorbent particles. The fibres can be natural or synthetic. According to another exemplary embodiment, the absorbent material is substantially fluffless. Typically, absorbent material refers to a material that is applied in bulk, i.e. a 3D absorbent material, i.e. not as a sheet. In some embodiments, the absorbent material may comprise more than 20 weight% superabsorbent particles or more than 40 weight% superabsorbent particles or more than 60 weight% superabsorbent particles, or more than 80 weight% superabsorbent particles. The absorbent material may also be fluffless, i.e. with substantially without cellulose fibres.

Also disclosed herein is an absorbent article comprising a liquid pervious topsheet, a liquid impervious backsheet, absorbent material positioned between the liquid pervious topsheet and the liquid impervious backsheet, and at least one of a capillary acceleration sheet and a top core wrap between the absorbent material and the liquid pervious topsheet; The absorbent material, typically comprising superabsorbent particles and optionally cellulosic fluff pulp, is arranged on a bottom core wrap such that one or more channels are formed, wherein less absorbent material per surface area is present in the one or more channels compared to an area around the one or more channels, wherein preferably substantially no absorbent material is present in the one or more channels. The bottom core wrap is attached to the top core wrap and/or the capillary acceleration sheet in one or more permanent attachment zones in the respective one or more

By providing one or more attachment zones each having a width and/or length which is smaller than a width and/or length of the corresponding channel, the absorbent material surrounding the channel can expand more easily into the channel area upon wetting whilst still maintaining a good liquid guiding and distributing structure at the level of the permanent attachment zone. Further, there is less risk of small amounts of absorbent material being unintendedly present in the one or more attachment zones by having each attachment zones at a distance of the edges delimiting the corresponding channel.

Preferably each permanent attachment zone includes all permanent attachment portions within the respective channel, i.e. outside of the permanent attachment zone no further permanent attachment bonds are present in the corresponding channel. Each permanent attachment zone may be either a continuous attachment zone or a discontinuous attachment zone including a plurality of bonding spots at a distance of each other. For a continuous attachment zone, the average width is the average of the width (measured in the transverse direction of the absorbent core) along its full length (along a longitudinal direction of the absorbent core) of the attachment zone. For a discontinuous attachment zone including a plurality of bonding spots at a distance of each other, the attachment zone may be defined as a polygon including adjacent peripheral bonding points or edges of the plurality of discrete bonding spots, and interconnecting said adjacent peripheral bonding points or edges through straight lines, wherein the average width (wb) of each permanent attachment zone is an average of the width of the polygon measured in the transverse direction of the absorbent core over the full length of the polygon.

A permanent attachment zone is an attachment zone which remain attached when wetted. This may be tested by applying 200 ml liquid substantially equally across the absorbent core and evaluating the attachment zone after 10 minutes. If the attachment zone remains intact, the attachment zone is a permanent attachment zone. More preferably, a permanent attachment zone is an attachment zone which remains intact after submerging the absorbent core in the liquid and waiting for 10 minutes. Preferably, the average width of each permanent attachment zone is less than 90% of the average width of the corresponding channel, preferably less than 80%, more preferably less than 70%, and more than 25% of the average width of the channel.

Preferably, the average length of each permanent attachment zone is less than 99%, more preferably less than 98% of the average length of the corresponding channel, e.g. less than 97% or even 95%, and/or more than 80% of the average length of the channel.

Preferably, each permanent attachment zone is an elongate attachment zone.

Preferably, each permanent attachment zone is centred with respect to the corresponding channel.

Preferably, in an area of the channel surrounding the permanent attachment zone no permanent bonds are present. In that manner, the absorbent material can easily expand into that surrounding area upon wetting of the absorbent material. Optionally, in an area of the channel surrounding the permanent attachment zone one or more semi-permanent attachment portions may be present. Such semi-permanent attachment portions are configured to loosen upon wetting of the absorbent article.

Preferably, a shortest distance between a periphery of each channel and a periphery of the corresponding attachment zone is larger than 2 mm, preferably larger than 3 mm, e.g. larger than 4 mm. In that way absorbent material may expand into a portion of the channel along the full periphery of the channel.

In an exemplary embodiment, the bottom core wrap is sealed in accordance with a sealing pattern to the top core wrap and/or to the capillary acceleration in the one or more permanent attachment zones, wherein preferably the sealing is realized by heat and/or pressure and/or ultrasonic energy. Optionally, the sealing pattern covers less than 70%, preferably less than 60%, more preferably less than 50%, even more preferably between 1 and 40% of the surface area of the one or more attachment zones. The sealing pattern may be a regular pattern, such as a line pattern or a pattern of dots. The sealing pattern may comprise a large number of distinct sealing areas, such as dots or lines, spread across the one or more attachment zones, wherein the large number is larger than 10. Optionally, the sealing pattern comprises a plurality of discrete elements, wherein each element has a first dimension in a first direction and a second dimensions in a direction perpendicular to the first direction, and wherein the first dimension is smaller than 2 mm, preferably smaller than 1.5 mm, more preferably smaller than 1 mm, e.g. between 0.1 and 0.7 mm.

Preferably, the average width (wc) of each channel is at least 7% of the width (w) of the absorbent core, preferably at least 10%. As explained above, the width of the absorbent core is measured in a transverse direction of the absorbent core, in an area where the respective one or more channels are present, between two end points where absorbent material is present, i.e. the width of the absorbent core is defined as the width of the absorbent material deposition area of the absorbent core. Thus, the top core wrap and/or the back core wrap may extend over a width which is larger than the width of the absorbent core, as they may be attached along a periphery where no absorbent material is present. In the area where the one or more channels are present, the width of the absorbent core may vary between a minimum width and a maximum width. Preferably the above specified criteria for the width of the channel is valid both for the minimum and the maximum width.

The width of the channel may be constant through substantially the whole length of the channel or may vary along its length. Preferably, the one or more channels are formed by one or more absorbent material-free zones within the absorbent material deposition area of the absorbent core, and the width of a channel is considered to be the width of the material-free zone, disregarding the possible presence of core wrap within the channel. Similarly, the width of the permanent attachment zone may be constant through substantially the whole length of the channel or may vary along its length.

Further disclosed herein is an absorbent article comprising a liquid pervious topsheet, a liquid impervious backsheet, absorbent material positioned between the liquid pervious topsheet and the liquid impervious backsheet, and a capillary acceleration sheet and/or a top core wrap between the absorbent material and the liquid pervious topsheet. The absorbent material, typically comprising superabsorbent particles and optionally cellulosic fluff pulp, is arranged on a bottom core wrap such that one or more channels are formed, wherein less absorbent material per surface area is present in the one or more channels compared to an area around the one or more channels, wherein preferably substantially no absorbent material is present in the one or more channels. The one or more channels are one or more elongate channels having a length measured in a longitudinal direction of the absorbent core, and each channel has a width measured in a transverse direction of the absorbent core, said width being at least 7% of the width of the absorbent core, preferably at least 10%.

By providing such wide channels, an excellent liquid guidance and distribution function is obtained.

Preferably, each channel is an elongate channel having a length, measured in a longitudinal direction of the absorbent core, which is at least four times the width of the channel.

Preferably, the bottom core wrap has a basis weight which is larger than the basis weight of the top core wrap and/or larger than the basis weight of the capillary acceleration sheet.

Any of the features described above to characterize the bottom core wrap and/or the top core wrap and/or the capillary acceleration sheet, may also be combined with the last two aspects described above.

According to another aspect there is provided a method for manufacturing an absorbent article. The method comprises the steps of providing a liquid pervious topsheet and a liquid impervious backsheet; arranging an absorbent material, comprising superabsorbent particles and optionally cellulosic fluff, between a bottom core wrap, on one side, and a top core wrap and a capillary acceleration sheet, on the other side, such that one or more channels are formed, wherein less absorbent material per surface area is present in the one or more channels compared to areas around the one or more channels, wherein preferably substantially no absorbent material is present in the one or more channels; and arranging the liquid pervious topsheet at the side of top core wrap and the capillary acceleration sheet and the liquid impervious backsheet at the side of the bottom core wrap, wherein the capillary acceleration sheet has the features described above, as defined in claim 17.

Preferably, the bottom core wrap is attached to the top core wrap and the capillary acceleration sheet by applying heat and/or pressure in the one or more permanent attachment zones according to a sealing pattern comprising a plurality of discrete elements.

Preferably, the bottom core wrap is attached to the top core wrap and the capillary acceleration sheet by applying more heat and/or pressure in each permanent attachment zone than in an area of the corresponding channel surrounding the permanent attachment zone. For example the heat and/or pressure in an area of the corresponding channel surrounding the permanent attachment zone may be set such that one or more semi-permanent attachment spots are created in this surrounding area.

Preferably, the arranging of the absorbent material comprises the steps of guiding a first sheet material along a rotating member, wherein a surface of said rotating member is provided with a pattern with suction zones and non-suction zones; wherein said non-suction zones are intended to form the one or more channels; applying an absorbent material on said first sheet material on the rotating member such that the suction zones are covered with absorbent material and substantially no absorbent material is present on the non-suction zones; and applying a second sheet material on top of the absorbent material on the first sheet material; and at least partially attaching said first sheet material to said second sheet material in the areas where substantially no absorbent material is present, such that one or more attachment zones in said one or more channels are formed. One of said first and second sheet material is a material for forming the capillary acceleration sheet and/or the top core wrap, and the other one is a material for forming the bottom core wrap.

Preferably, the attaching is done by applying pressure and/or heat and/or ultrasonic energy on the bottom core wrap and the capillary acceleration sheet material and/or the top core wrap material in the areas where substantially no absorbent material is present. For example, a sealing pattern as described above may be used for the attaching. Optionally the sealing with a sealing pattern may be combined with applying an adhesive between the capillary acceleration sheet material and/or top core wrap, on the one hand, and the bottom core wrap on the other hand.

Any of the features described above in connection with the absorbent article are also applicable for embodiments of the method.

### BRIEF DESCRIPTION OF FIGURES

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1A is a schematic cross-section of an exemplary embodiment of an absorbent article with an absorbent core according to the prior art; figures 1B and 1C are cross-sections of exemplary embodiments of an absorbent article with an absorbent core according to the invention; figure 1D is a detailed view of the bottom core wrap of figure 1C;
Figures 2A-2B are schematic top views of the absorbent core of the exemplary embodiments of figures 1A-1B, respectively;
Figures 3-10 are schematic top plan views of exemplary embodiments of a diaper;
Figure 11 is a schematic top plan view of another exemplary embodiment of a diaper with four channels; and
Figure 12 is a perspective view of an exemplary embodiment of a diaper in the wetted state;
Figure 13 illustrates schematically an exemplary embodiment of a method and apparatus for manufacturing an absorbent article;
Figures 14A-E illustrate an example of a set of sealing bars having a sealing pattern;
Figure 15 is a schematic top plan views of exemplary embodiment of an absorbent core; and
Figure 16A and 16B illustrate an embodiment of a channel, as seen in a cross section, in a dry state and in a wet state, respectively;
Figures 17-21 are schematic top plan views of exemplary embodiments of a channel with a permanent attachment zone.

### DESCRIPTION OF EMBODIMENTS

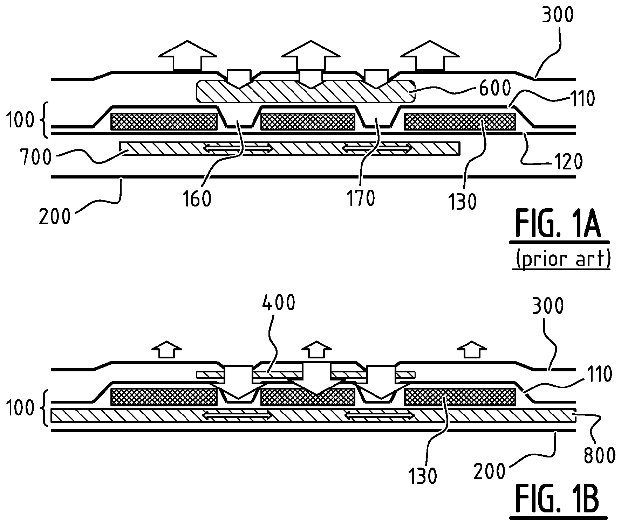

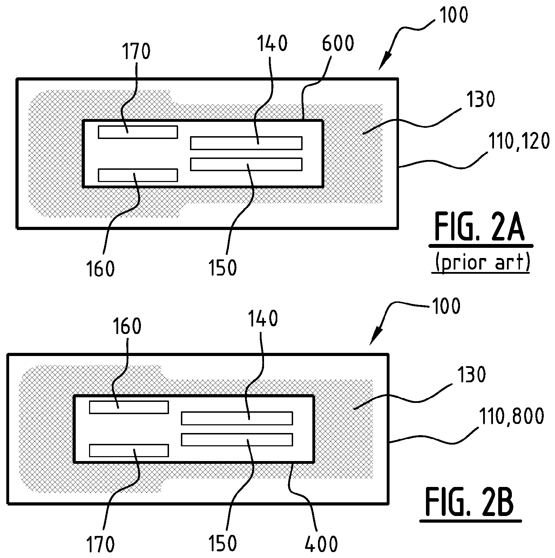

Figures 1A and 2A show an exemplary embodiment of an absorbent article with an absorbent core 100 with multiple channels 140, 150, 160, 170, according to the prior art. The upper side of the absorbent article generally has a topsheet 300 that can be liquid pervious (not shown in figure 2A for clarity reasons). The lower side has a backsheet 200 (not shown in figure 2A for clarity reasons) that can generally be liquid impervious and is joined with the topsheet 300 at the edges of the absorbent article (not shown). In some embodiments of adult incontinence products, the topsheet 300 and the backsheet 200 are not joined at the edges. An absorbent core 100 is positioned between the topsheet 300 and the backsheet 200. The absorbent core 100 comprises absorbent material 130 arranged between a bottom core wrap 120 and a top core wrap 110, in such a manner that one or more channels 140, 150, 160, 170 are formed, wherein less absorbent material 130 per surface area is present in the one or more channels 140, 150, 160, 170 compared to an area around the one or more channels. An acquisition and distribution layer (ADL) 600 is provided at the top of the absorbent core 100, beneath the topsheet 300. The topsheet 300, the backsheet 200, and the absorbent core 100 can be assembled in a variety of well-known configurations. For example, the topsheet 300 and the backsheet 200 can be joined to each other by adhesive, by heat bonding, pressure bonding, ultrasonic bonding, dynamic mechanical bonding, a crimp seal, or by any other suitable securing method. In prior art embodiment the ADL 600 is typically a relatively thick layer (basis weight above 50 g/m²) and comprises a coarse fibre. Further, a liquid-impermeable, either hydrophilic or hydrophobic, distribution or wicking layer 700 is included between the absorbent core 100 and the backsheet 200. This distribution or wicking layer 700 helps to wick and transport liquids and having a capability to disperse the liquid over the surface of said wicking layer from the less absorbent area's (e.g. saturated) to the more absorbent area's (e.g. unsaturated). Due to the specific absorbent capacity of the distribution or wicking layer 700, the liquid in the one or more channels will be drawn up into the distribution layer and will spread out throughout the rest of the distribution layer. In that way, the distribution, transport and absorption of the liquid can be improved.

Figures 1B and 2B illustrate schematically a first exemplary embodiment of the invention. The absorbent article comprises a liquid pervious topsheet 300, a liquid impervious backsheet 200, an absorbent core 100 comprising absorbent material 130, and a capillary acceleration sheet 400 between the absorbent core 100 and the liquid pervious topsheet 300. The absorbent core 100 is positioned between the liquid pervious topsheet 300 and the liquid impervious backsheet 200. The absorbent material 130 is arranged between a bottom core wrap 800 and a top core wrap 110, such that one or more channels 140, 150, 160, 170 are formed, wherein less absorbent material 130 per surface area is present in the one or more channels 140, 150, 160, 170 compared to an area around the one or more channels, wherein preferably substantially no absorbent material is present in the one or more channels 140, 150, 160, 170. The top core wrap 110, the bottom core wrap 800 and the capillary acceleration sheet 400 may have any one or more of the features disclosed above in the summary.

The top core wrap 110 is attached to the bottom core wrap 800 at least in a portion of the one or more channels 140, 150, 160, 170, and preferably the capillary acceleration sheet 400 is attached to the top core wrap 110 in at least a portion of the one or more channels 140, 150, 160, 170. This is not shown in figure 1B but the skilled person understands that the capillary acceleration sheet 400 may be attached to the top core wrap 110 at the level of the channels 160 and/or 170 and/or 140 and/or 150.

The capillary acceleration sheet 400 may take on any suitable shape. For example, the capillary acceleration sheet 400 may follow more or less the contours of the absorbent core 100. In other forms, the capillary acceleration sheet may comprise a rectangular shape regardless of the shape of the absorbent core 100. In some forms, the capillary acceleration sheet may be longer in length than the absorbent core 100 or shorter than the absorbent core 100. In other forms, the capillary acceleration sheet 400 may be positioned in a specific location on the absorbent core 100.

To provide for softness next to the body, the topsheet 300 can be formed from a soft, smooth, flexible, porous material that is non-irritating to the user's skin. The topsheet 300 is permeable to the body fluids to be collected by the absorbent article. Generally, topsheets for absorbent articles can be made from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibres), synthetic fibres (e.g., polymeric fibres such as polyester, polypropylene, or polyethylene fibres) or from a combination of natural and synthetic fibres.

Hydrophobic topsheets or topsheets with a low hydrophilicity have a reduced tendency to allow fluids to pass back through and rewet the wearer's skin. Thus, the surface of the topsheet that is in contact with the body remains dry, thereby reducing body soiling and creating a more comfortable feel for the wearer.

Example topsheets are topsheets selected from typical nonwoven forming approaches such as spunbonded, through-air bonded (TAB), carded, hydro-entangled, needled, or high loft nonwoven topsheets, and apertured 2-dimensional or 3-dimensional film topsheets. Spunbonded or through-air bonded (TAB) webs or through-air bonded carded (TABC) webs are preferred. Preferably, the through air bonded carded web comprises polyethylene/polypropylene bi-component staple fibres. "Bonded carded web" refers to webs that are made from staple fibres that are sent through a combing or carding unit, which breaks apart and aligns the staple fibres in the machine direction to form a generally machine direction-oriented fibrous nonwoven web. This web may then be drawn through a heated drum, creating bonds throughout the fabric without applying specific pressure (through air bonding process). The "Through air bonded carded web", TABCW material provides a relatively low density, lofty web.

Preferably, the topsheet has a basis weight between 10 and 30 g/m², preferably between 15 and 25 g/m². Preferably, the fibres of the topsheet have an average fineness between 1-3 dtex.

Lofty apertured formed film topsheets, with appreciable topsheet texture (nubs, micro-texture or with filament-like protrusions on the body-facing surface that can trap bodily discharges and hinder low fluid flows towards the body) that may be hydrophobic or hydrophilic in nature, can also be used.

It is possible and advantageous to bond the topsheet 300 directly or indirectly to the underlying capillary acceleration sheet 400. These layers may be bonded by any known bonding means, such as slot gluing, spiral gluing, fusion point bonding, sealing according to a sealing pattern, or otherwise attached.

Optionally, a distribution layer may be present between the absorbent core 100 and the capillary acceleration sheet 400. However, preferably no distribution layer is present between the capillary acceleration sheet 400 and the absorbent core 100. The function of a distribution layer is to spread the insulting fluid liquid over a larger surface within the article so that the absorbent capacity of the absorbent core 100 can be more efficiently used. Typically the distribution layer is made of a nonwoven material based on synthetic or cellulosic fibres and having a relatively low density.

The capillary acceleration sheet 400 is positioned at the body-facing side of the absorbent core 100, between the topsheet 300 and the absorbent core 100 of the absorbent article, and preferably in close proximity or even in good contact with the body-facing side of the absorbent core 100. The use of a capillary acceleration sheet 400 in combination with the one or more channels 140, 150 in the absorbent core 100 leads to a very good distribution of fluids from a discharge area to the entire absorbent core 100 and an increased dryness feeling at the level of topsheet 300, and this shortly after liquid insult.

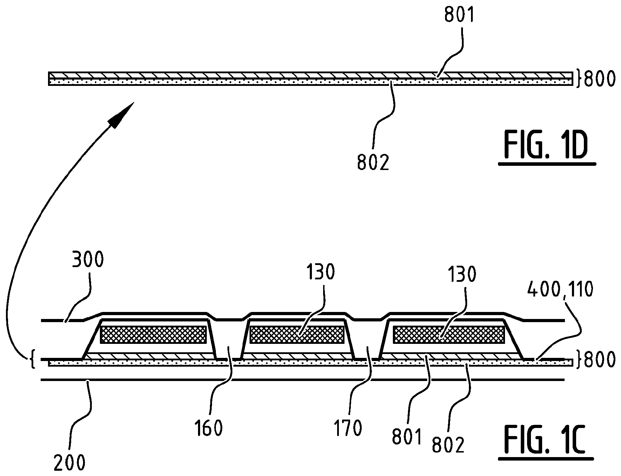

Figure 1D illustrates in more detail a possible bottom core wrap 800 comprising a first layer 801 and a second layer 802, wherein said first layer has a first density between 20 and 150 kg/m3 and said second layer has a second density between 100 and 400 kg/m3, said second density being higher than the first density. As shown in the embodiment of figure 1C, the first layer 801 is closer to the absorbent material 130 than said second layer 802 which faces the backsheet 200. The absorbent article of figure 1C may comprise a capillary acceleration sheet 400 and/or a top core wrap 110. As shown the capillary acceleration sheet 400 and/or the top core wrap 110 may be attached through the first layer 801 to the second layer 802 of the bottom core wrap 800 in the channel areas 160, 170. Optionally also the topsheet 300 and/or the backsheet 200 may be attached at least in a portion of the channel areas 160, 170 to the bottom core wrap 800 (not shown). Further, the bottom core wrap 800 is attached along a periphery to the bottom core wrap 110 and/or the capillary acceleration sheet 400. The bottom core wrap may be glued and/or sealed to the top core wrap 110 and/or the capillary acceleration 400 at least in a portion of the one or more channels.

The bottom core wrap 800 may consist of a single sheet. Preferably, the bottom core wrap 800 is in direct contact with the superabsorbent particles of the absorbent material 130. Preferably, a difference between the first and the second density is higher than 20 kg/m3, preferably higher than 30 kg/m3 more preferably higher than 40 kg/m2, even more preferably between 50 and 150 kg/m3, most preferably between 60 and 140 kg/m3. Preferable, the second density is selected such that it forms a barrier for the superabsorbent particles.

Preferably, the bottom core wrap 800 is a through-air bonded non-woven. In an example, the first layer 801 is a through-air-bonded carded non-woven and the second layer 802 is a spunbond layer.

The first layer 801 has a first basis weight and the second layer 802 has a second basis weight, said second basis weight being lower than said first basis weight. The difference between the first layer and the second layer may be larger than 1 g/m2, preferably larger than 2 g/m2, more preferably larger than 5 g/m2. The first layer may have a first basis weight between 15 and 80 g/m2, preferably between 25 and 80 g/m2, and/or the second layer may have a second basis weight between 8 and 35 g/m2, preferably between 8 and 25 g/m2. Preferably, the first layer and/or the second layer comprise more than 90weight% of synthetic fibres, preferably more than 95weight% of synthetic fibres.

In an example, the first layer comprises polyester fibres, and the second layer comprises any one of the following fibres or a combination thereof: polyethylene, polyester, copolyester, polypropylene, polyactic acid (PLA).

The attachment between the top core wrap and the bottom core wrap 800 may be a continuous or discontinuous attachment. For example, the top core wrap may be attached to the bottom core wrap 800 along a major portion of the one or more channels 140, 150, 160, 170, with optionally one or more unattached portions being present between attached portions. More generally, the one or more channels 140, 150, 160, 170 may comprise one or more permanent attachment portions which remain attached after wetting and/or one or more semi-permanent attachment portions which detach after wetting and/or one or more unattached portions. An unattached portion or a semi-permanent attachment portion allows for an increased swelling of the absorbent material 130 after the one or more channels 140, 150, 160, 170 have fulfilled their function of distributing the liquid. Indeed, wet absorbent material 130 is allowed to extend/swell into a portion of the channel area 140, 150, 160, 170 in case of an unattached or detached portion.

In the embodiment of figures 1B and 2B, the capillary acceleration sheet 400 covers entirely the one or more channels 140, 150, 160, 170. Such an arrangement leads to a substantial improvement in the distribution of fluids over the complete absorbent core 100. Nevertheless, other arrangements also provide a good distribution of liquids. For example, the capillary acceleration sheet 400 may only partially overlap with the one or more channels 140, 150, 160, 170 or may be located between two longitudinal channels or between two lateral channels. For example, the one or more channels may extend in a front portion of the absorbent core 100 beyond a front edge of the capillary acceleration sheet 400 and/or in a rear portion of the absorbent core 100, beyond a rear edge of the capillary acceleration sheet 400. Because the capillary acceleration sheet 400 does not cover a front and/or rear end of the one or more channels, fluid in-flow and/or fluid out-flow may be increased.

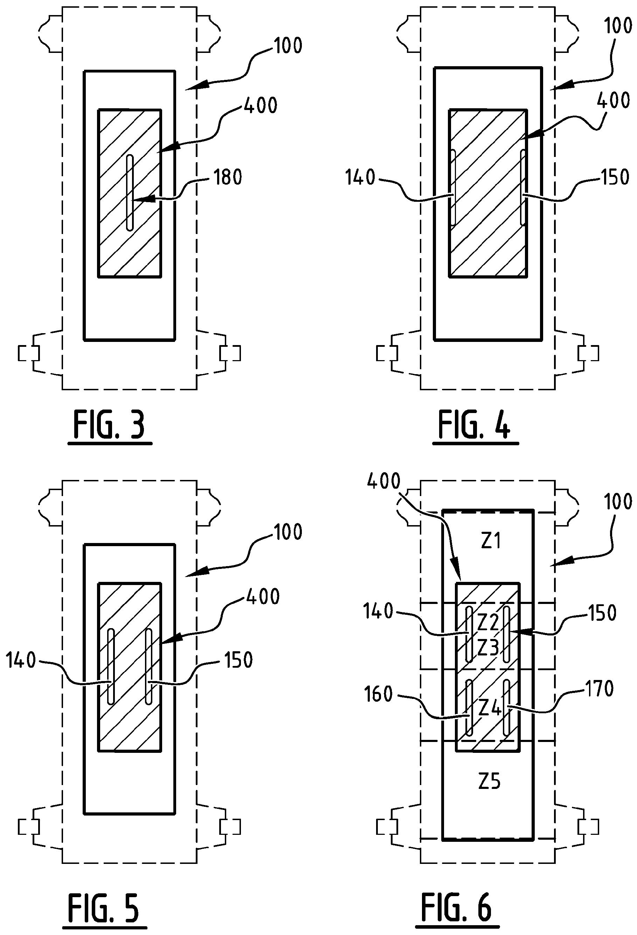

Now a series of variants will be illustrated with reference to figures 3-12.

Figure 3 illustrates an exemplary embodiment of an absorbent article with an absorbent core 100 with a single central channel 180 extending in a longitudinal direction of the absorbent core 100. In figure 3 the capillary acceleration sheet 400 is shown to extend over only a portion of the surface area of the absorbent core 100, but it will be understood that it can also extend over the entire surface area of the absorbent core 100.

Preferably, the channel 180 extends over at least 20% of the length of the absorbent core 100, more preferably at least 30%, even more preferably at least 40%. Preferably, the channel 180 extends, seen in the transverse direction of the absorbent core, over the transverse distance which is at least 1 mm, preferably at least 3 mm, more preferably at least 4 mm, even more preferably at least 5 mm, most preferably at least 6 mm. The length 180 of the channel may be larger than 30 mm, preferably larger than 40 mm, more preferably larger than 50 mm. In the channel 180 the capillary acceleration sheet 400 may be attached to the bottom core wrap 800 through one or more permanent or semi-permanent attachment portions. The semi-permanent portions may be configured to release after having been in contact with liquid whilst said permanent portions may be configured not to release after having been in contact with liquid. It is noted that different types of semi-permanent attachment portions may be used in the same absorbent article so as to achieve a gradual loosening of the attachments upon wetting. The absorbent material in the absorbent core 100 preferably comprises cellulosic fluff pulp and/or superabsorbent particles. However, the absorbent material may be substantially fluffless.

Preferably, substantially no absorbent material is present in the channel 180. A position and/or shape of the channel 180 may be indicated by means of a distinguishable colour and/or coloured pattern. E.g., a position and/or shape of the channel 180 may be indicated by means of a printed ink layer. The distinguishable colour and/or coloured pattern may be provided on at least one of the topsheet, the top core wrap, the capillary acceleration sheet 400, the backsheet and the bottom core wrap.

According to a preferred embodiment, outside of the channel 180 the absorbent core has a maximum thickness and the channel 180 extends through at least 90 % of the maximum thickness of the absorbent core, more preferably through substantially 100% of the thickness of the absorbent core 100 such that in the first and second attachment zone substantially no absorbent material is present between the capillary acceleration sheet 400 and the bottom core wrap.

According to a preferred embodiment, the attachment between the capillary acceleration sheet 400 and the bottom core wrap is any one of the following or a combination thereof: pressure bonding, thermal bonding, sonic bonding, chemical bonding, adhesive. The attachment may be a direct or indirect attachment. E.g. an adhesive and/or other intermediate material may be inserted between the capillary acceleration sheet 400 and the bottom core wrap.

Figure 4 and figure 5 illustrate exemplary embodiments with two channels 140, 150. In figures 4 and 5 the capillary acceleration sheet 400 is shown to extend over only a portion of the surface area of the absorbent core 100, but it will be understood that it can also extend over the entire surface area of the absorbent core 100.

The absorbent core 100 has a first and second longitudinal edge and a first and second transverse edge and the channels comprise 140, 150 are elongate channels extending from a crotch region in the direction of the first and second transverse edge. The channels 140, 150 may comprise one or more attachment zones where the capillary acceleration sheet 400 and/or the top core wrap is attached to the back core wrap sheet. Preferably the one or more attachment zones are formed by any one of the following or a combination thereof: pressure bonding, thermal bonding, sonic bonding, chemical bonding, adhesive. Preferably, a width of the channels 140, 150, seen in a transverse direction of the absorbent core 100, is at least 5 mm, preferably at least 6 mm, more preferably at least 8 mm, most preferably at least 9 mm, e.g. between 9 and 20 mm or between 10 and 20 mm or between 10 and 15 mm or between 12 and 15 mm. The channels 140, 150 may comprise any one or more of the following: one or more permanent attachment zones, one or more semi-permanent attachment zones configured to release after having been in contact with liquid, one or more unattached zones.

Preferably, each channel has a width measured in a transverse direction of the absorbent core, said width being at least 5% of the width of the absorbent core 100, preferably at least 7%, e.g. at least 10%, e.g. at least 15% or even at least 20%. For example, the channel width may be between 9 and 20 mm and the width of the absorbent material deposition area may be between 80 and 200 mm, e.g. between 90 and 150 mm. Preferably, the width of each channel is less than 30% of the width of the absorbent core, e.g. less than 25%. By having wider channels, the risk of getting fluff material in the one or more channels may be reduced.

The channels 140, 150 extend next to each other and are each extending in the direction of a first and/or second transverse edge. Preferably, the capillary acceleration sheet 400 at least partially covers both channels 140, 150. In figure 4 the capillary acceleration sheet 400 partially covers the channels 140, 150, whilst in figure 5 the channels are fully covered. Preferably, the distance between the channels 140, 150 is between 10 mm and 50 mm, preferably between 15 mm and 30 mm; and the length of the first and the second channel is larger than 60 mm, preferably larger than 70 mm.

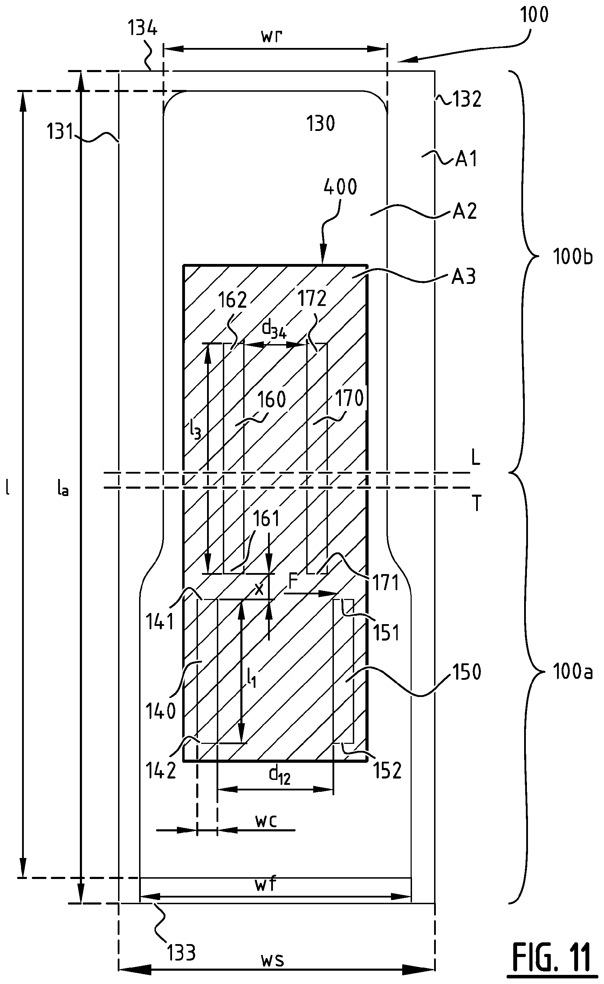

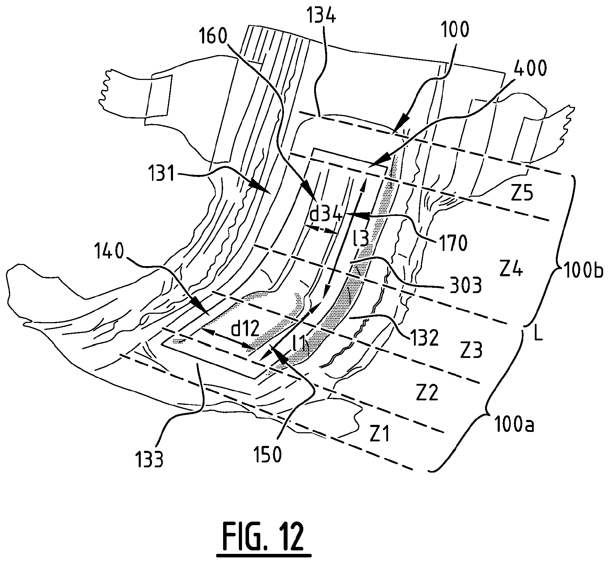

The absorbent core 100 has a front portion extending at one side of a transverse crotch line and a rear portion extending at the other side of the transverse crotch line. In figures 3-10, the front portion corresponds with an upper portion of the absorbent core 100, whilst in figures 11 and 12 the front portion 100a corresponds with the lower portion of the absorbent core 100.

In figures 4 and 5, the illustrated channels 140, 150 are straight channels but it will be understood that the channels may also be curved channels, wherein the distance between the channels increases toward the front transverse edge and/or towards the rear transverse edge of the absorbent core 100.

Figure 6, figure 11 and figure 12 illustrate an embodiment with four channels: a first channel 140, a second channel 150, a third channel 160, and a fourth channel 170. In figures 6, 11 and 12, the capillary acceleration sheet 400 is shown to extend over only a portion of the surface area of the absorbent core 100, but it will be understood that it can also extend over the entire surface area of the absorbent core 100.

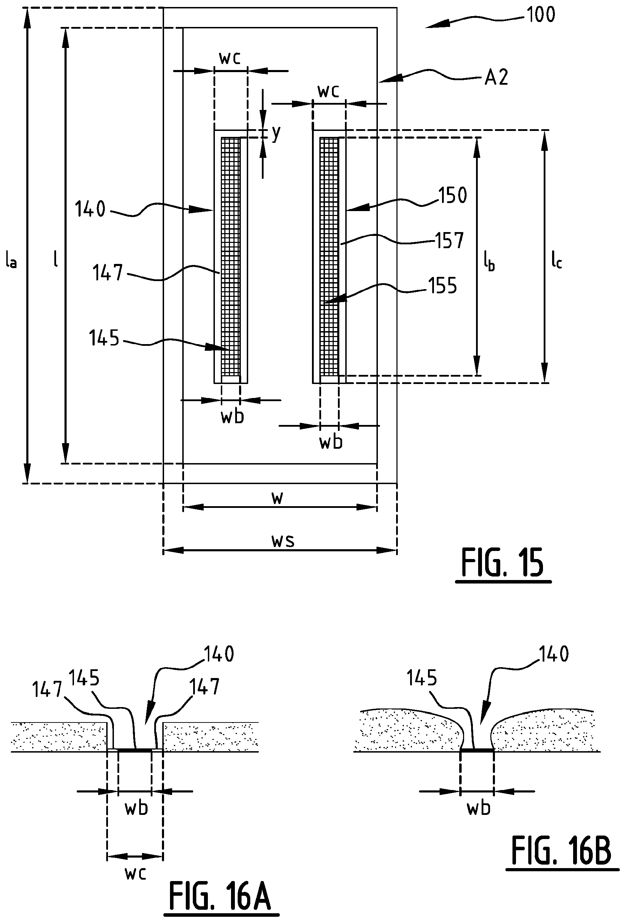

The third and fourth channel 160, 170 are located at a distance of each other and at a distance of the first and second channel 140, 150. As illustrated, the capillary acceleration sheet 400 may covers all four channels 140, 150, 160, 170. Preferably, as illustrated in figures 11 and 12, the distance between the first and the second channel 140, 150 is different from the distance between the third and the fourth channel 160, 170. Preferably, the first and second channel 140, 150 extend at least in the front portion 100a of the absorbent core 100, and the third and fourth channel 160, 170 extend at least in the rear portion 100b of the absorbent core 100, wherein preferably the distance between the first and the second channel 140, 150 is bigger than the distance between the third and the fourth channel 160, 170. Optionally, the first channel 140 is connected to the third channel 160 through a first semi-permanent attachment zone and the second channel 150 is connected to the fourth attachment zone 170 through a second semi-permanent attachment zone. Preferably, the first, second, third and fourth channels comprise permanent attachment zones, more preferably permanent attachment zones as illustrated below in connection with Figure 15.

Preferably, the first and second channel 140, 150 extend longitudinally between respective ends 141, 142, 151, 152, and the third and fourth channel 160, 170 extend longitudinally between respective ends 161, 162, 171, 172. A first end 141 of the first channel 140 may be located at a distance x of a first end 161 of the third channel 160, with absorbent material extending between the ends 141 and 161. Similarly, a first end 151 of the second channel 150 may be located at a distance x of a first end 171 of the fourth channel 170, with absorbent material extending between the ends 151 and 171.

As illustrated in Figure 11, the absorbent core 100 has a front edge 133, a rear edge 134, and opposite longitudinal side edges 131, 132 which define an area A1. The absorbent material 130 is deposited in an area A2 within the area A1. In the present application the length and width of the absorbent core refer to the length l and width wf, wr of the area A2. The area A2 may be slightly smaller than the area A1.

Optionally, a position and/or shape of one or more channels 140, 150, 160, 170 is indicated by means of a distinguishable colour and/or coloured pattern, e.g. by a printed ink layer, wherein preferably the distinguishable colour and/or coloured pattern is provided on at least one of the topsheet, the capillary acceleration sheet 400, the backsheet and the bottom core wrap. Preferably, the channels 140, 150, 160, 170 and the capillary acceleration sheet 400 are arranged symmetrically with respect to a longitudinal centre line of the bottom core wrap.

A first distance between the first and the second channel 140, 150 may be at least 5%, preferably at least 10% bigger, even more preferably at least 20% bigger than a second distance between the third and the fourth channel 160, 170. This difference may be optimized in function of the desired used. For example, for male persons the difference may be bigger.

Seen in a projection on the longitudinal direction of the absorbent core 100, the first and the second channel 140, 150 may extend over a length 11 which is less than the length l3 of the third and fourth channel 160, 170. To fit better to the body the third and fourth channel 160, 170 which are closer to each other may be longer to extend over a longer part of the crotch region, for example the third and fourth channel 160, 170 may extend both in the front and the rear portion 100a, 100b of the absorbent core 100. Preferably, the first and the second channel 140, 150 extend over a length 11 which is at least 5% less, more preferably at least 10% less than the length l3 of the third and fourth channel 160, 170. Preferably the first and the second channel 140, 150 extend over a length l1 which is at least 25%, more preferably at least 35%, even more preferably at least 45% of the length l3 of the third and fourth channel 160, 170.

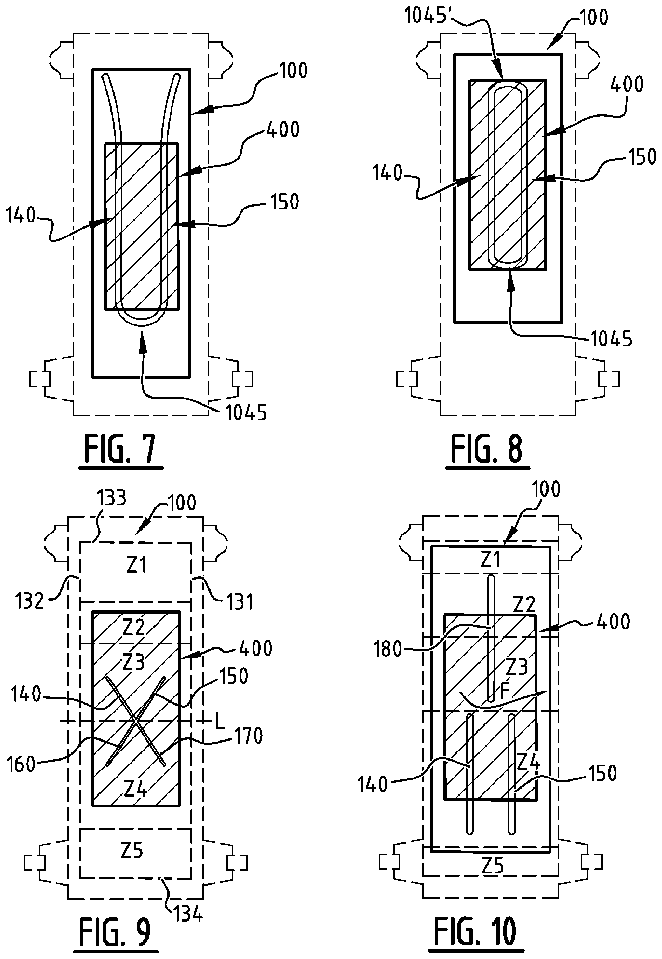

Figures 7 and 8 illustrate an absorbent article comprising an absorbent core 100 having a first and second longitudinal edge and a front and rear transverse edge. The absorbent core is provided with two interconnected elongate channels 140, 150 extending next to each other from a crotch region in the direction of the front and/or rear transverse edge and at least one connecting channel 1045, 1045' connecting the first channel with the second channel. By providing a first and a second elongate channel which are interconnected by at least one connecting channel, upon wetting of the absorbent core, liquid can flow from the first elongate channel to the second elongate channel and vice versa, improving the liquid distribution, whereupon the liquid can be absorbed by the absorbent material. In figures 7 and 8, the capillary acceleration sheet 400 is shown to extend over only a portion of the surface area of the absorbent core 100, but it will be understood that it can also extend over the entire surface area of the absorbent core 100.

In figure 7 the at least one connecting channel comprises a rear connecting channel 1045 which connects a rear end portion of the first channel 140 to a corresponding rear end portion of the second channel 150. In figure 8 both a rear connecting channel 1045 and a front connecting channel 1045' which connects a front end portion of the first channel to a corresponding front end portion of the second channel, are provided. In that manner a good distribution is obtained in the front portion and/or in the rear portion. Especially for a male person, it may be desirable to have a front connecting channel.

The absorbent core 100 has a transverse crotch line dividing the absorbent core in a front portion and a rear portion on either side of the transverse crotch line. Preferably, the front connecting channel is located in the front portion and/or the rear connecting channel is located in the rear portion.

Preferably, the absorbent core 100 is substantially symmetrical with respect to a longitudinal center axis. For example, the channel area 140, 150, 1045, 1045' may be substantially V-shaped or U-shaped or O-shaped, wherein the V-shape or U-shape is arranged such that it is symmetrical with respect to the longitudinal center axis of the absorbent core.

In an exemplary embodiment, the first and second channels comprise permanent attachment zones which remain attached upon wetting, or semi-permanent attachment zones configured to release after having been in contact with liquid for a predetermined period of time, wherein said predetermined period of time is preferably smaller than 30 s. In a preferred embodiment the at least one connecting channel 1045, 1045' comprises any one or more of: a permanent attachment portion which remains attached upon wetting, a semi-permanent attachment portion configured to release after having been in contact with liquid for a predetermined period of time, wherein said predetermined period of time is preferably smaller than 30s, and unattached portion. Attachment portion/zone refers to a portion/zone where the capillary acceleration sheet 400 and/or the top core wrap is attached to the bottom core wrap.

In an exemplary embodiment, the first channel and the second channel are substantially parallel and extend in a longitudinal direction of the absorbent core 100. In another embodiment, see figure 7, the first and second channel 140, 150 may diverge in the direction of the front edge of the absorbent core 100.

In an exemplary embodiment, the largest distance between the first and the second channel in the transverse direction is between 15 and 70% of the width of the absorbent core, more preferably between 20 and 50%; wherein preferably the largest distance between the first and the second channel in the transverse direction is between 10 mm and 100 mm, more preferably between 20 mm and 80 mm, even more preferably between 30 mm and 70 mm.

Figure 9 illustrates an embodiment with an X-shaped channel area. In figure 9, the capillary acceleration sheet 400 is shown to extend over only a portion of the surface area of the absorbent core 100, but it will be understood that it can also extend over the entire surface area of the absorbent core 100.

The absorbent core 100 has a first and second longitudinal edge and a first and second transverse edge, and a longitudinal center line dividing the absorbent core in a first longitudinal portion and a second longitudinal portion on either side of the longitudinal center line, and a transverse crotch line dividing the absorbent core in a front portion and a rear portion on either side of the transverse crotch line. The absorbent core is provided with a plurality of channels 140, 150, 160, 170 comprising a first channel 140, 170 and second channel 150, 160 extending next to each other from a crotch region in the direction of the front and rear transverse edge. The first channel 140, 170 crosses the longitudinal center line in a first crossing point, from the first longitudinal portion to the second longitudinal portion; and the second elongate channel 150, 160 crosses the longitudinal center line in second crossing point, from the second longitudinal portion to the first longitudinal portion. The first and second crossing point may be the same point (as in figure 9) or a different point, and may be located in the front portion or in the rear portion or on the transverse crotch line between connecting the front portion to the rear portion. By providing a first and a second channel which are crossing the longitudinal center line, upon wetting of the absorbent core 100, liquid is guided in the first and/or second elongate channel from left to right and/or from right to left, respectively, whilst flowing towards the crotch region or away from the crotch region, improving the liquid distribution, whereupon the liquid can be absorbed by the absorbent material. Further, by making the first and second channels cross the longitudinal center line, the channels may be longer compared to similar channels extending parallel to the longitudinal center line, resulting in a larger liquid distribution zone.

Preferably, the first and/or second crossing point are located at a distance of the transverse crotch line. For example, the first and/or second crossing point may be located in a front portion. In that way the position of the first and/or second can be optimized e.g. in function of whether the absorbent article is intended for a male or female person. However, in other embodiments, the first and/or second crossing point may be located on the transverse crotch line. When at a distance of the transverse crotch line, preferably, the distance between the first and/or second crossing point and the transverse crotch line is larger than 1% of the length of the absorbent core, preferably larger than 2%, even more preferably larger than 3%.

In certain embodiments with multiple first crossing points and/or multiple second crossing points, these multiple first and/or second crossing points may be located a different distances of the transverse crotch line, and may comprise e.g. two first crossing points, one in the front portion and one in the rear portion, and two second crossing points, one in the front portion (optionally corresponding with the first crossing point in the front portion), and one in the rear portion (optionally corresponding with the first crossing point in the rear portion).

Preferably, the first elongate channel 140, 170 extends both in the front portion and in the rear portion; and the second elongate channel 150, 160 extends both in the front portion and in the rear portion. In that manner a good liquid distribution from left to right and from front to rear can be obtained. Preferably, the first elongate and the second elongate channels are arranged symmetrically with respect to the longitudinal center line of the absorbent core. Preferably, a maximum distance between the first and the second elongate channel is between 15 and 70% of the width of the absorbent core, more preferably between 20 and 50%.

In an exemplary embodiment a maximum distance between the first and the second channel in the front portion is different from a maximum distance between the first and the second channel in the rear portion. In that manner the liquid distribution zone may be better adapted to the type of person wearing the absorbent article. For example, for a male person, a maximum distance between the distance between the first and the second channel near a front transverse edge may be larger than a maximum distance between the first and the second channel in a rear portion.

Preferably, the length of the first and second channel is larger than 10% of the length of the absorbent core, more preferably larger than 30%, even more preferably larger than 50%.

In an exemplary embodiment, the first and second channel together form a substantially X-shaped zone. Optionally the legs of the "X" may be interrupted to create one or more bridging zones.

Figure 10 illustrates an embodiment of an absorbent article with an absorbent core 100 comprising three channels 140, 150, 180. In figure 10, the capillary acceleration sheet 400 is shown to extend over only a portion of the surface area of the absorbent core 100, but it will be understood that it can also extend over the entire surface area of the absorbent core 100.

The channels comprise a first and second channel 140, 150 similar to the first and second channel of figure 6, and a third channel 180 extending from the crotch region in the direction of the second transverse edge, wherein seen in a projection on a transverse direction the third channel 180 is located between the first and the second channel 140, 150. By having a first and a second channel in the front or rear portion and a third channel in the rear or front portion, respectively, it is possible to tailor the absorbent article to the wearer. For example, for a male person the first and second channel may be in the front portion and the third channel may be in the rear portion, whilst for a female person the first and second channel may be in the rear portion and the third channel in the front portion. Further it is possible to optimize the difference between the front and the rear portion for obtaining a unisex absorbent article.

Seen in a projection on the longitudinal direction of the absorbent core, the first and the second channel 140, 150 may extend over a length which is less than the length of the third channel 180. To fit better to the body the third channel 180 may be longer to extend over a longer part of the crotch region, for example the third channel may extend both in the front and the rear portion of the absorbent core. Preferably, the first and the second channel extend over a length which is at least 5% less, more preferably at least 10% less than the length of the third channel. Preferably the first and the second channel extend over a length which is at least 25%, more preferably at least 35%, even more preferably at least 45% of the length of the third channel.

In all embodiments described above, and in particular in the embodiments of Figures 3-12, preferably, each channel has a width wc (indicated in Figure 11) measured in a transverse direction of the absorbent core, said width being at least 5% of the width (varies between wf and wr in the embodiment of Figure 11) of the absorbent core 100, preferably at least 7%, e.g. at least 10%, e.g. at least 15% or even at least 20%. For example, the channel width may be between 9 and 20 mm and the width of the absorbent material deposition area A2 (indicated in Figure 11) may be between 80 and 200 mm, e.g. between 90 and 150 mm. Preferably, the width of each channel is less than 30% of the width of the absorbent core, e.g. less than 25%. By having wider channels, the risk of getting fluff material in the one or more channels may be reduced.

The width of the absorbent core is measured in a transverse direction of the absorbent core, in an area where the respective one or more channels are present, between two end points where absorbent material is present, i.e. the width of the absorbent core is defined as the width of the absorbent material deposition area A2 of the absorbent core 100. Thus, the top core wrap sheet and/or the back core wrap sheet may extend in an area A1 over a width ws (as indicated in Figure 11) which is larger than the width of the absorbent core and a length la which is larger than the length of the absorbent core, as they may be attached along a periphery where no absorbent material is present. In the area where the one or more channels are present, the width of the absorbent core may vary between a minimum width and a maximum width (varies between wf and wr in the embodiment of Figure 11; not indicated in the other figures but may vary in a similar way or may be constant or may vary in a different way), and preferably the above specified minimum width of each channel is valid both for the minimum and the maximum width of the absorbent core. The absorbent material deposition area A2 may thus have a width which varies e.g. between a value above 100 mm and a value below 100 mm. Preferably the difference is at least 5 mm. For example, a width wf of the absorbent material deposition area measured at a front end of the absorbent core 100 may be above 100 mm, or even above 105 mm and a width wr measured at a rear end of the absorbent core 100 may be below 100 mm, or even below 95 mm.

The width wc of the channel may be constant through substantially the whole length of the channel (as shown in the Figures) or may vary along its length. When the width varies along the length, then the above specified width percentages and values apply for the average width of the respective channel. Preferably, the one or more channels are formed by one or more absorbent material-free zones within the absorbent material deposition area of the absorbent core, and the width of a channel is considered to be the width of the material-free zone, disregarding the possible presence of core wrap within the channel.

The absorbent core periphery, which may be the periphery of the top/bottom core wrap, may define any suitable shape, such as a "T," "Y," "hour-glass," or "dog-bone" shape, for example. A "T shape may have a larger width in the front portion than in the rear portion of the absorbent core. An absorbent core periphery having a generally "dog bone" or "hour-glass" shape may taper along its width towards the middle or "crotch" region of the core. In this way, the absorbent core may have a relatively narrow width in an area of the absorbent core intended to be placed in the crotch region of an absorbent article.

For all embodiments described above, preferably, the one or more channels cover together at least 20%, preferably at least 30 %, more preferably at least 40%, more preferably at least 50%, e.g. at least 60%, or at least 70%, or 80% or at least 90% of a total length l of the absorbent core. The covered length may be realized with a single channel or with a combination of two or more channels. This will allow a good distribution over the entire absorbent core as well as a good formation of the channels/embankments and will give the absorbent article a tub-shape upon swelling of the absorbent core. The length of the absorbent core is defined as the length l of the absorbent material deposition area A2 indicated in Figure 11. The length la of the bottom core wrap and/or of the top core wrap may be slightly larger than the length l of the absorbent material deposition area A2.

The absorbent article 100 may further comprise a wetness indicator preferably placed between two channels and/or in one or more channels and/or between a channel and an edge of the absorbent core. The wetness indicator may change appearance when contacted with liquid, e.g. the wetness indicator may be configured to generate a colour change signal that changes appearance when contacted with liquid. The wetness indicator may comprise a composition that changes appearance when contacted with liquid, in particular a composition comprising a pH indicator and/or a water soluble dye. The composition may comprise a stabilizer, a colorant, and a matrix.

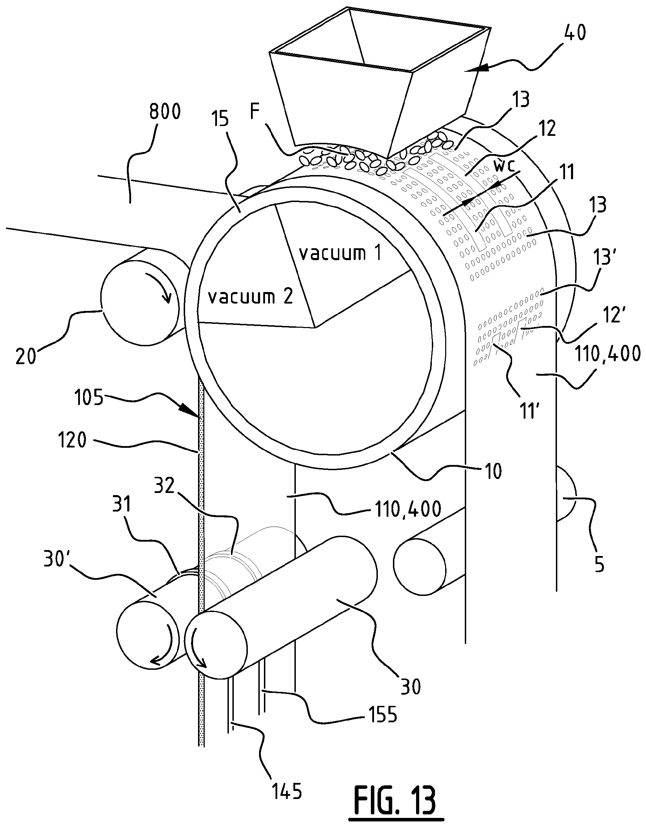

Figure 13 illustrates an embodiment of a method for manufacturing an absorbent article. The method comprises in a first step guiding a first sheet material 110, 400 along an optional guide roller 5, and further along a rotating member 10, wherein a surface 15 of said rotating member 10 is provided with a pattern with suction zones 13, 13' and non-suction zones 11, 12; 11', 12'. The first sheet material 110, 400 is shown in a transparent manner to reveal the suction and non-suction zones of the rotating member 10. The suction zones 13, 13' may be provided with holes, and the non-suction zones 11, 12; 11', 12' are formed of closed material. For example, the non-suction zones 11, 12; 11', 12' may be provided with inserts. The inserts 11, 12; 11', 12', may have a trapezoidal cross section. Figure 10 shows an insert pattern with four non-suction zones per absorbent core, but it will be understood that the number of inserts depends on the number of channels to be realized. The inserts may be fixed e.g. with screws on the rotating member 10. At an inner area of the rotating member 10 a vacuum is applied, see VACUUM 1. The non-suction zones 11, 12; 11', 12' comprise at least a first elongate zone 11, 11' and a second elongate zone 12, 12' extending in a circumferential direction of the rotating member 10. In a second step an absorbent material F is applied via a hopper 40 on said first sheet material 110, 400 on the rotating member 10 such that the suction zones 13, 13' are covered with absorbent material and substantially no absorbent material is present on the non-suction zones 11, 12; 11', 12'. In a third step a second sheet material 800 is applied on top of the absorbent material on the first sheet material 110, 400, e.g. using a further rotating member 20. The first sheet material 110, 400 may be a combination of the capillary acceleration sheet material 400 and the top core wrap material 110, and the second sheet material 800 may be bottom core wrap. However, in another embodiment, the second sheet material is a combination of the top core wrap material and the capillary acceleration sheet material, and the first sheet material may be the bottom core wrap. In a fourth step the first sheet material 110 is attached to the second sheet material 800 at least in the areas where substantially no absorbent material is present, and such that one or more channels 140, 150, 160, 170 are formed. The attaching may be done by applying pressure and/or heat and/or ultrasonic energy on the sandwich formed by the first sheet material 110, 400 and on the second sheet material 800, especially in the one or more channels where substantially no absorbent material is present, e.g. by a rotating member 30 and/or opposite rotating member 30' which is provided with at least a first and a second seal rib or bar 31, 32 dimensioned for applying pressure and heat in the areas where substantially no absorbent material is present in order to create the channels 140, 150, 160, 170, respectively.

The capillary acceleration sheet 140, the bottom core wrap 800 and the top core wrap 110 may have any one of the features described above or described in the summary.

While the above-described method of manufacturing absorbent articles has good results, the first sheet material and the second sheet material may be additionally attached using a binder, such as glue, to strengthen the bond between the first and second sheet material. For example, gluing may be performed as described in patent EP 3 453 368 B1 in the name of the applicant, which is included herein by reference.

The capillary acceleration sheet material 400 may also be first laminated, e.g. adhered, to a topsheet material, and the combination of the capillary acceleration sheet 400 and the topsheet may then be arranged against the top core wrap 110, wherein optionally the capillary acceleration sheet 400 may be glued to the top core wrap 110.

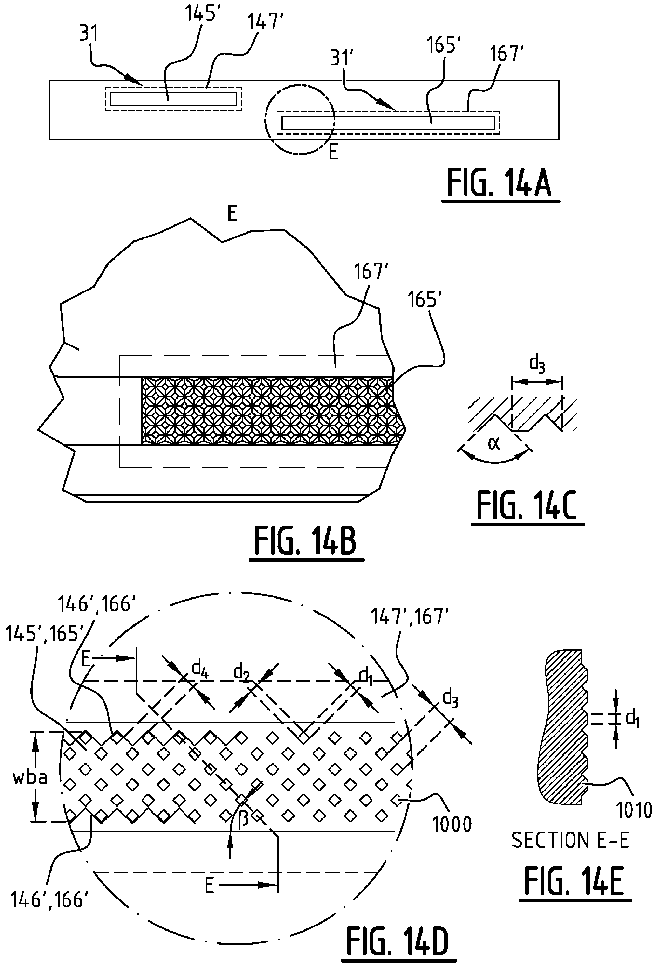

Figures 14A-E illustrate an example of a set of sealing bars 31, 31' having a first sealing pattern. The sealing bars 31, 31' may be intended to seal portions of e.g. channels 160 and 140. The sealing bars 31, 31' comprise respective sealing portions 145', 165' intended to seal permanent attachment zones 145, 165 of channels 160 and 140. These permanent attachment zones 145, 165 may be smaller than the channel areas 140, 160, see further. Optionally further respective sealing portions 147', 167' intended to create semi-permanent attachment zones 147, 167 around the permanent attachment zones 145, 165 may be provided. Such further respective sealing portions 147', 167' may have a height which is lower than the height of sealing portions 145', 165' such that less pressure is applied. In addition or alternatively the temperature of the further respective sealing portions 147', 167' may be controlled during sealing such that it is lower than the temperature of sealing portions 145', 165'. Such measures will allow to generate a permanent attachment zone surrounded by a semi-permanent attachment zone which is such that it releases upon wetting.

It will be understood that one or more sealing bars may be used in accordance with the one or more channels to be sealed. A detail of the sealing pattern is visible in the top view of figure 14B and in figure 14D which illustrates the areas where sealing will take place. Here the sealing pattern is a regular pattern of square shaped dots 1000. Figure 14E shows a cross section and illustrates that the dots are achieved by protrusions 1010. Each protrusion 1010 preferably has a flat top surface forming a dot 1000 of the sealing pattern, and an inclined peripheral surface. If an absorbent particle were to be present near the flat top surface it can easily migrate in a recess delimited by the inclined peripheral wall.

Preferably, the sealing pattern (i.e. the total surface area of the dots 1000) covers less than 80%, preferably less than 70%, more preferably less than 60%, even more preferably less than 50%, most preferably between 1 and 50% of the surface area of the one or more channels. For example, the sealing pattern covers between 1 and 50% of the total surface area of the one or more channels, or between 1 and 40%, or between 1 and 30%, or between 1 and 25%, or between 2 and 25%, or between 3 and 25%, or between 4 and 25%. Such sealing pattern may also be used in the optional further sealing portions 147', 167'.

Such sealing pattern can provide a good resistance against the swell forces generated by liquid being captured in the superabsorbent particles by hydrogen bonds, but the sealing pattern equally can provide resistance against dry and wet superabsorbent particles trying to penetrate through the capillary acceleration sheet and through the sealing pattern after as well as during the sealing step of the manufacturing process. In that manner, any risk of dry or wet SAP particles coming into contact with sensitive skin upon wearing is avoided or reduced. Further, by having a reduced surface area that is being sealed in accordance with a pattern, any particles remaining in the one or more channel zones can easily migrate to a non-sealing area so that the risk of creating holes in the one or more channel zones is reduced or avoided.

Although a pattern of dots 100 is illustrated, the skilled person understands that other sealing patterns are possible, such as a line pattern e.g. a grid, etc. Also, the dots may have any shape, e.g. round, polygonal, etc. The line pattern may comprise one or more sets of parallel lines. When a first set of parallel lines and a second set of parallel lines are included, the lines of the first set may be oriented at a non-zero angle with respect to the lines of the second set.

Preferably, the sealing pattern comprises a large number of distinct sealing areas (here dots 1000) spread across the one or more channels. Preferably, the large number is larger than 10, more preferably larger than 20.

Preferably, the sealing pattern comprises a plurality of discrete elements (here dots 1000), and each discrete element has a first dimension d1 in a first direction and a second dimension d2 in a direction perpendicular to the first direction. The first dimension d1 is smaller than 2 mm, preferably smaller than 1.5 mm, more preferably smaller than 1 mm, e.g. between 0.1 and 0.7 mm or between 0.2 and 0.7 mm, or between 0.3 and 0.6 mm, e.g. 0.5 mm as illustrated. Preferably also the second dimension d2 is smaller than 2 mm, preferably smaller than 1.5 mm, more preferably smaller than 1 mm, e.g. between 0.1 and 0.7 mm or between 0.2 and 0.7 mm, or between 0.3 and 0.6 mm, e.g. 0.5 mm as illustrated. However, also line patterns are possible in which case the second distance d2 may be much longer than the first distance d1.

Preferably, the distance d4 between adjacent discrete elements (here dots 1000) is smaller than 10 mm, preferably smaller than 5 mm, more preferably smaller than 2 mm, e.g. between 0.1 and 2.0 mm or between 0.2 and 1.5 mm, or between 0.3 and 1.0 mm, e.g. 0.7 mm as illustrated. Preferably, the pitch distance d3 (=d1+d4) of the dot pattern is smaller than 10 mm, preferably smaller than 5 mm, more preferably smaller than 2 mm, e.g. 1.2 mm as illustrated.

Preferably, the angle α between adjacent inclined surfaces of the protrusions 1010 is between 60 and 120 degrees, more preferably between 70 and 110 degrees, even more preferably between 80 and 100 degrees.

Preferably, plurality of discrete elements (here dots 1000) is aligned along lines oriented at an angle β with respect to a longitudinal direction of the absorbent core. Preferably, the angle β is between 20 and 160 degrees, more preferably between 30 and 150 degrees.

Figure 15 illustrates an absorbent core 100 with two channels 140, 150, preferably free of absorbent material, which are arranged within an absorbent material deposit area A2. The absorbent core 100 has and a bottom core wrap, and a top core wrap and/or a capillary acceleration sheet. The top core wrap, bottom core wrap and capillary acceleration sheet may have any one of the features described above. The bottom core wrap is attached to the top core wrap and/or the capillary acceleration sheet, in two permanent attachment zones 145, 155 within the respective channels 140, 150. Seen in a transverse direction of the absorbent core, an average width wb of each permanent attachment zone 145, 155 is smaller than an average width (wc) of the respective channel 140, 150. Further, seen in a longitudinal direction of the absorbent core 100, an average length lb of each attachment zone is smaller than an average length lc of the corresponding channel. Preferably, the average width wb of each permanent attachment zone 145, 155 is less than 90% of the average width wc of the respective channel 140, 150, preferably less than 80% of wc, more preferably less than 70% of wc, and preferably more than 25% of wc.

In Figure 15 the widths wb and wc are shown to be constant, but it will be understood that those widths may be variable and that the average width wb and wc is an average of the width along the length of the attachment zone and channel, respectively.

Each permanent attachment zone 145, 155 may be a continuous attachment zone or may consist of a plurality of discrete permanent bonding spots. In the latter case, in the context of this application an imaginary attachment zone is defined by drawing a polygon shape around the plurality of bonding spots, said polygon including adjacent peripheral bonding points or edges of the plurality of discrete bonding spots, and interconnecting said adjacent peripheral bonding points or edges through straight lines£. The average width of each permanent attachment zone is an average of the width of the polygon measured in the transverse direction of the absorbent core over the full length of the polygon. An example is illustrated in Figure 14D, where the permanent attachment zone 145', 165' is delimited by saw tooth lines 146', 166', which will result in an average width wba as indicated.

In an area 147, 157 of the channel 140, 150 surrounding the permanent attachment zone 145, 155 preferably no permanent bonds are present. However, optionally, a semi-permanent attachment may be present in this surrounding area 147, 157, as was also explained above in connection with Figures 14A-14D.

Preferably, the average width (wc) of each channel is at least 5% of the average width (w) of the absorbent core, more preferably at least 7%, e.g. at least 10%. The average width wc of each channel may be e.g. between 10 and 20 mm. The average width wb of each permanent attachment zone 145, 155 may be e.g. between 5 mm and 10 mm.

Preferably, each channel 140, 150 is an elongate channel, e.g. a straight channel or a curved channel, having length lc, measured in a longitudinal direction of the absorbent core, which is at least four times the width wc of the channel.

Preferably, the average length (lb) of each permanent attachment zone is at least 5 mm less than the average length (lc) of the corresponding channel, preferably at least 7 mm less.

Preferably, the attachment zone 145, 155 is substantially centred within the corresponding channel 140, 150 so that on either side of the attachment zone 145, 155 there is a channel portion which does not contain permanent bonds, allowing absorbent material to expand in those channel portions upon wetting. This is further illustrated in Figures 16A and 16B which illustrate a channel 140 with permanent attachment zone 145 before and after wetting, respectively.

Preferably, a shortest distance y between a periphery of each channel 140, 150 and a periphery of the corresponding attachment zone 145, 155 is larger than 2 mm, more preferably larger than 3 mm, e.g. larger than 4 mm.

The width wc is the width of the channel which preferably corresponds with an area where substantially no absorbent material is present. Thus, the width wc corresponds substantially with the width of the non-suction zones 11, 12, 11', 12' illustrated in Figure 13 above.

Preferably, the permanent attaching is done by applying adhesive and/or pressure and/or heat and/or ultrasonic energy on the bottom core wrap, on the one hand, and the top core wrap and/or the capillary acceleration sheet, on the other hand, in a portion of the channels. For example, a sealing pattern as described above may be used for the attaching. Optionally the sealing with a sealing pattern may be combined with applying an adhesive between the capillary acceleration sheet material and/or top core wrap, on the one hand, and the bottom core wrap on the other hand.

Although Figure 15 illustrates an embodiment with two channels, the described features may be equally applied in any one of the embodiments described above with one channel or with more than two channels.

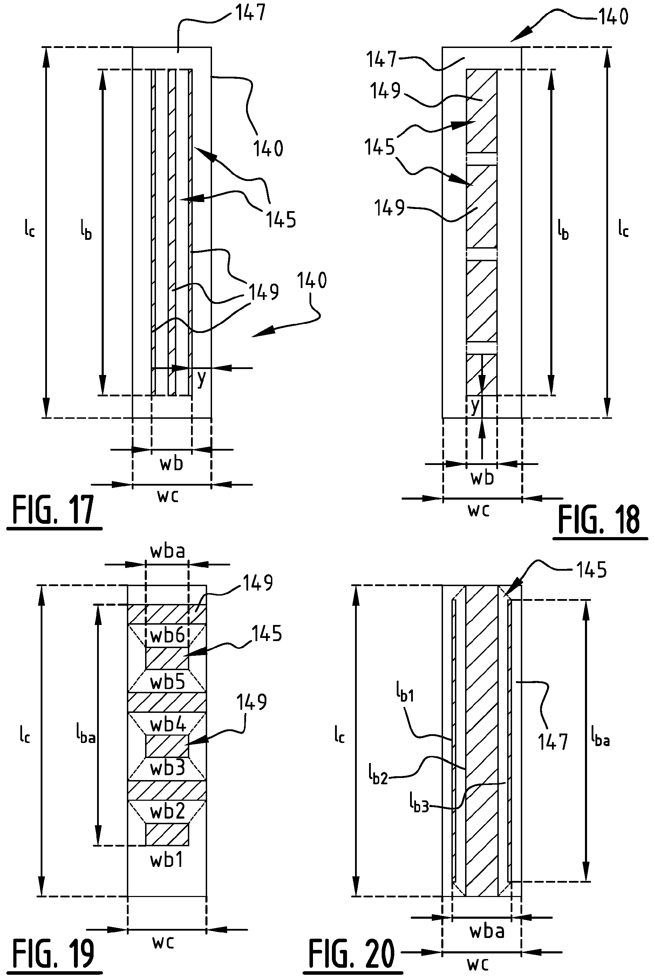

Figures 17-21 illustrate further exemplary embodiments of a channel 140 with a permanent attachment zone 145 comprising a plurality of distinct permanent bonding spots 149.

In the embodiment of Figure 17, the plurality of distinct permanent bonding spots is a plurality of substantially parallel lines 149 having a length lb which is smaller than the length lc of the channel. Further the average width wb of the attachment zone 145 (here a rectangle including the lines 149 and contacting the most peripheral edges of the lines 149 so that the width thereof is constant) is smaller than the channel width wc. Preferably, the width (wb) of the permanent attachment zone 145 is less than 80% of the width (wc) of the corresponding channel, preferably less than 70%, e.g. less than 60%. Preferably, the length (lb) of the permanent attachment zone 145 is at least 5 mm less than the average length (lc) of the channel, preferably at least 7 mm less. Preferably, the average length (lb) of the permanent attachment zone 145 is more than 80% of the average length (lc) of the channel. Preferably, in an area 147 of the channel 140 surrounding the permanent attachment zone 145 no permanent bonds are present. Optionally one or more semi-permanent attachment zones may be present in the area 147. Preferably, a shortest distance (y) between a periphery of the channel 140 and a periphery of the corresponding attachment zone 145 is larger than 2 mm, preferably larger than 3 mm, e.g. larger than 4 mm.

In the embodiment of Figure 18, the plurality of distinct permanent bonding spots is a plurality of longitudinally aligned rectangles 149 having a constant width wb which is smaller than the width wc of the channel 140. Further the length lb of the attachment zone 145 (here a rectangle 145 including the rectangles 149 and contacting the most peripheral edges of the rectangles 149 so that the length of the large rectangle 145 is constant) is smaller than the channel length lc. Preferably, the width (wb) of the permanent attachment zone 145 is less than 80% of the width (wc) of the corresponding channel, preferably less than 70%, e.g. less than 60%. Preferably, the length (lb) of the permanent attachment zone 145 is at least 5 mm less than the length (lc) of the channel, preferably at least 7 mm less. Preferably, the length (lb) of the permanent attachment zone 145 is more than 80% of the length (lc) of the channel. Preferably, in an area 147 of the channel 140 surrounding the permanent attachment zone 145 no permanent bonds are present. Optionally one or more semi-permanent attachment zones may be present in the area 147. Preferably, a shortest distance (y) between a periphery of the channel 140 and a periphery of the corresponding attachment zone 145 is larger than 2 mm, preferably larger than 3 mm, e.g. larger than 4 mm.

In the embodiment of Figure 19, the plurality of distinct permanent bonding spots is a plurality of longitudinally aligned rectangles 149 having variable widths wb1-wb5 some of which are smaller than the width wc of the channel 140. Further the average length lba of the attachment zone 145 (here a polygon 145 including the rectangles 149 and connecting the most peripheral edges of the rectangles 149) is smaller than the channel length lc. Preferably, the average width wba of the permanent attachment zone 145 is less than 80% of the width (wc) of the channel, preferably less than 70%, e.g. less than 60%. Preferably, the average length (lba) of the permanent attachment zone 145 is at least 5 mm less than the length (lc) of the channel, preferably at least 7 mm less. Preferably, the average length (lba) of the permanent attachment zone 145 is more than 80% of the length (lc) of the channel.

In the embodiment of Figure 20, the plurality of distinct permanent bonding spots is a plurality of substantially parallel lines 149 having variable lengths lb1, lb2, lb3 some of which are smaller than the length lc of the channel. Further the average width wba of the attachment zone 145 (here a polygon including the lines 149 and interconnecting the most peripheral edges of the lines 149) is smaller than the channel width wc. Preferably, the average width (wba) of the permanent attachment zone 145 is less than 80% of the width (wc) of the corresponding channel, preferably less than 70%, e.g. less than 60%. Preferably, the average length (lba) of the permanent attachment zone 145 is at least 5 mm less than the average length (lc) of the channel, preferably at least 7 mm less. Preferably, the average length (lba) of the permanent attachment zone 145 is more than 80% of the average length (lc) of the channel.

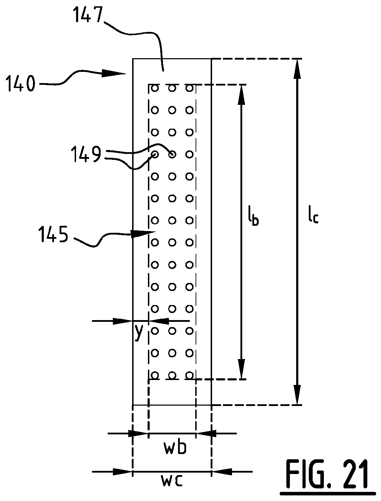

In the embodiment of Figure 21, the plurality of distinct permanent bonding spots is a plurality of dots 149, e.g. round dots arranged in a grid having a length lb which is smaller than the length lc of the channel. Further the width wb of the attachment zone 145 (here a rectangle including the dots 149 and contacting the most peripheral points of the peripheral dots 149) is smaller than the channel width wc. Preferably, the width (wb) of the permanent attachment zone 145 is less than 80% of the width (wc) of the corresponding channel, preferably less than 70%, e.g. less than 60%. Preferably, the length (lb) of the permanent attachment zone 145 is at least 5 mm less than the average length (lc) of the channel, preferably at least 7 mm less. Preferably, the average length (lb) of the permanent attachment zone 145 is more than 80% of the average length (lc) of the channel. Preferably, in an area 147 of the channel 140 surrounding the permanent attachment zone 145 no permanent bonds are present. Optionally one or more semi-permanent attachment zones may be present in the area 147. Preferably, a shortest distance (y) between a periphery of the channel 140 and a periphery of the corresponding attachment zone 145 is larger than 2 mm, preferably larger than 3 mm, e.g. larger than 4 mm.

As illustrated in all embodiments of Figures 17-21, in the context of the application, when a plurality of discrete permanent bonding spots is included, the permanent attachment zone as defined here has a polygon shape, said polygon including adjacent peripheral bonding points or edges of the plurality of discrete bonding spots, and interconnecting said adjacent peripheral bonding points or edges through straight lines. Stated differently, the adjacent left edges or points are interconnected through straight lines, adjacent rear edges or points are interconnected through straight lines, adjacent right edges or points are interconnected through straight lines and adjacent rear edges or points are interconnected through straight lines, such that a polygon is formed which includes all permanent bonding spots and touches the most peripheral edges or points thereof.

### Glossary

As used in the present application, the following terms have the following meanings:
"A", "an", and "the" as used herein refers to both singular and plural referents unless the context clearly dictates otherwise. By way of example, "an edge barrier" refers to one or more than one edge barrier.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.

"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.

"Absorbent core" as used herein refers to a three-dimensional part of the absorbent structure, comprising liquid-absorbing material, useful to permanently absorb and/or retain bodily exudates. "Absorbent component" as used herein refers to a structural constituent of an absorbent article, e.g., a piece of an absorbent core, such as one of multiple pieces in a multi-piece absorbent core. "Absorbent element" as used herein refers to a part of a functional constituent of an absorbent structure, e.g., an acquisition layer, a dispersion layer, core layer or a release structure formed of a material or materials having particular liquid handling characteristics suitable for the specific function.

"Absorbent fibrous polymer material" as used herein refers to an absorbent polymer material which is in threadlike from such as fibers, filaments, and the like so as to be less flowable in the dry state than particulates.

"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.

"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).

"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).

"Absorbent particulate polymer material" as used herein refers to an absorbent polymer material which is in particulate form such as powders, granules, flakes and the like so as to be flowable in the dry state.

"Absorption" as used herein refers to the process by which a liquid is taken up within a material.

"Absorption rate" as used herein refers to the rate of absorption of liquid, i.e. the amount of liquid which is absorbed per unit of time, typically by an absorbent component, element and/or absorbent layer of the absorbent article, structure and/or core.

"Acquisition layer", "acquisition region", "acquisition surface" or "acquisition material" and the like as used herein refer to the layer overlying the absorbent core having a faster liquid uptake and/or distribution capability.

"Absorbency" is the ability of a material to take up fluids by various means including capillary, osmotic, solvent, chemical and/or other action.

"Adult incontinence garment" as used herein refers to absorbent articles intended to be worn by incontinent adults, for absorbing and containing bodily exudates.

"Adhesion" as used herein refers to the force that holds different materials together at their interface.

"Adhesive" as used herein refers to a material, which may or may not be flowable in solution or when heated, that is used to bond materials together.

"Adsorption" as used herein refers to the process by which a liquid is taken up by the surface of a material.

"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".

"Apparent density", "density" as used herein refers to the basis weight of the sample divided by the caliper with appropriate unit conversions incorporated therein. Apparent density used herein has the unit g/cm3.

"Attach", "attached" and "attachment" as used herein are synonymous with their counterparts of the terms "fasten", "affix", "secure", "bind", "join" and "link".

"Baby diaper" as used herein refers to absorbent articles intended to be worn by children, for absorbing and containing bodily exudates which the user draws up between the legs and fastens about the waist of the wearer.

"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.

"Back region" as used herein refers to the portion of an absorbent article or part thereof that is intended to be positioned proximate the back of a wearer.

"Backing" as used herein refers to a web or other material that supports and reinforces the back of a product.

"Basis weight" is the weight per unit area of a sample reported in grams per square meter, g/m2 or gsm.

"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.

"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.

"Bond strength" as used herein refers to the amount of adhesion between bonded surfaces. It is a measure of the stress required to separate a layer of material from the base to which it is bonded. "Capillary action", "capillarity", or "capillary motion" and the like as used herein are used to refer to the phenomena of the flow of liquid through porous media.

"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.

"Cellulose fibers" as used herein refers to naturally occurring fibers based on cellulose, such as, for example cotton, linen, etc; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.

"Cluster" or the like as used herein refers to an agglomeration of particles and/or fibers.

"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.

"Cohesion" as used herein refers to the resistance of similar materials to be separated from each other.

"Compartment" as used herein refers to chambers, cavities, pockets and the like.

"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.

"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.

"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.

"Cross direction (CD)", "lateral" or "transverse" and the like as used herein are used interchangeably and refer to a direction which is orthogonal to the longitudinal direction and includes directions within ±45° of the transversal direction.

"Curing" as used herein refers to a process by which resins, binders or plastics are set into or onto fabrics, usually by heating, to cause them to stay in place; the setting may occur by removing solvent or by cross-linking so as to make them in soluble.

"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.

"Dispersion layer", "dispersion region", "dispersion surface" or "dispersion material" and the like as used herein refer to the layer overlying the absorbent core having a faster liquid uptake and dispersion capability.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

"Dry strength" as used herein refers to the strength of ajoint determined in dry state conditions, immediately after drying under specified conditions or after a period of conditioning in the standard laboratory atmosphere.

"Essentially cellulose free", "substantially fluffless" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.

"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.

"Fabric" as used herein refers to a sheet structure made from fibers, filaments and/or yarns. "Feminine hygiene garments" as used herein refer to absorbent hygiene articles intended to be worn by woman, for absorbing and containing body exudates.

"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.

"Fluff pulp" or "Pulp fluff" as used herein refers to wood pulp specially prepared to be drylaid. The fibers can be either natural or synthetic or a combination thereof.

"Front region" as used herein refers to the portion of an absorbent article or part thereof that is intended to be positioned proximate the front of a wearer.

"Garment facing layer" as used herein refers to elements of the chassis that form the outer surface of the absorbent article, such as the backsheet, the side panels, the waist fasteners, and the like, when such elements are present.

"Heat activated adhesive" as used herein refers to a dry adhesive that is rendered tacky or fluid by application of heat or heat and pressure to the assembly.

"Heat sealing adhesive" as used herein refers to a thermoplastic adhesive which is melted between the adherent surfaces by heat application to one or both of the adjacent adherent surfaces.

"High loft" as used herein refers to general term of low density, thick or bulky fabrics.

"Hot-melt adhesive" as used herein refers to a solid material that melts quickly upon heating, then sets to a firm bond upon cooling; used for almost instantaneous bonding.

"Hydrophilic" as used herein refers to having an affinity for being wetted by water or for absorbing water.

"Hydrophobic" as used herein refers to lacking the affinity for being wetted by water or for absorbing water.

"Immobilization layer" as used herein refers to a layer able to be applied to the absorbent polymer material or absorbent polymer material area with the intent to gather, bond and/or immobilize absorbent material and/or absorbent layer.

"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.

"Knitting" as used herein refers to the technique for interlocking loops of fibers with needles or similar devices.

"Layer" refers to identifiable components of the absorbent article, and any part referred to as a "layer" may actually comprise a laminate or combination of several sheets or webs of the requisite type of materials. As used herein, the term "layer" includes the terms "layers" and "layered."

"Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration.

"Machine direction (MD)", "longitudinal" and the like as used herein are used interchangeably and refer to a direction running parallel to the maximum linear dimension of the structure and includes directions within ±45° of the longitudinal direction.

"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

"Mass flow" as used herein refers to the flow of a liquid from one absorbent element or component to another absorbent element or component by channel flow action.

"Mechanical bonding" as used herein refers to a method of bonding fibers by entangling them. This can be achieved by needling, stitching with fibers or by the use of high-pressure air or water jets and the like.

"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or nonrefastenable bonds (e.g., seam, weld, adhesive, cohesive bond, fastener, etc.). A pant may be preformed anywhere along the circumference of the article (e.g., side fastened, front waist fastened).

"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.

"Rear" as used herein refers to the portion of an absorbent article or part thereof that is intended to be positioned proximate the back of the wearer.

"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.

"Resin" as used herein refers to a solid or semisolid polymeric material.

"Thermobonding" as used herein refers to a method of bonding fibers by the use of heat and/or high-pressure.

"Thermoplastic" as used herein refers to polymeric materials that have a melting temperature and can flow or be formed into desired shapes on the application of heat at or below the melting point.

"Ultrasonic" as used herein refers to the use of high frequency sound to generate localized heat through vibration thereby causing thermoplastic fibers to bond to one another.

"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.

"Wearer facing layer" as used herein refers to elements of the chassis that form the inner surface of the absorbent article, such as the topsheet, the leg cuffs, and the side panels, etc., when such elements are present.

"Weaving" as used herein refers to the process of interlacing two or more sets of yarns at right angles to form a fabric; a web of fibers produced by weaving is herein referred to as a "woven".

"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.

"Wet burst strength" is a measure of a layer's ability to absorb energy, when wet and subjected to deformation normal to the plane of the web.

"Wet strength" as used herein refers to the strength of a joint determined immediately after removal from a liquid in which it has been immersed under specified conditions of time, temperature and pressure. The term is commonly used in the art to designate strength after immersion in water.

"Wetlaying" as used herein refers to the forming a web from an aqueous dispersion of fibers by applying modified paper making techniques; a web of fibers produced by wetlaying is herein referred to as a "wetlaid".

"Wood pulp" as used herein refers to cellulosic fibers used to make viscose rayon, paper and the absorbent cores of products such as feminine hygiene garments, baby diapers and pants and adult incontinence garments.

"X-y dimension" as used herein refers to the plane orthogonal to the thickness of the article, structure or element. The x- and y-dimensions correspond generally to the width and length, respectively, of the article, structure or element.

"Z-dimension" as used herein refers to the dimension orthogonal to the length and width of the article, structure or element. The z-dimension corresponds generally to the thickness of the article, structure or element.

## Claims

1. An absorbent article comprising a liquid pervious topsheet (300), a liquid impervious backsheet (200), an absorbent core (100) positioned between the liquid pervious topsheet and the liquid impervious backsheet, said absorbent core comprising a bottom core wrap (120, 800), a top core wrap (110) and absorbent material (130) between the bottom core wrap and the top core wrap, and a capillary acceleration sheet (400) between the absorbent core and the liquid pervious topsheet (300);
wherein the absorbent material (130) comprises superabsorbent particles and optionally cellulosic fluff pulp, wherein the absorbent material is arranged such that one or more channels (140, 150, 160, 170, 180) are formed, wherein less absorbent material per surface area is present in the one or more channels compared to an area around the one or more channels, wherein preferably substantially no absorbent material is present in the one or more channels, wherein the capillary acceleration sheet (400) is a through-air-bonded nonwoven having a basis weight between 20 and 30 gsm.

2. The absorbent article of claim 1, wherein the capillary acceleration sheet (400) has been subjected to a hydrophilic spin finish treatment.

3. The absorbent article of any one of the previous claims, wherein the one or more channels are one or more elongate channels having a length measured in a longitudinal direction of the absorbent core, and wherein each channel has a width measured in a transverse direction of the absorbent core, said width being at least 5% of the width of the absorbent core, preferably at least 7%, e.g. at least 10%, and less than 30%.

4. The absorbent article of any one of the previous claims, wherein the bottom core wrap (120, 800) consists of a single sheet.

5. The absorbent article of any one of the previous claims, wherein the bottom core wrap (120, 800) comprises a through-air bonded non-woven, wherein preferably the bottom core wrap consists of a through-air bonded non-woven.

6. The absorbent article of any one of the previous claims, wherein the bottom core wrap (120, 800) has a basis weight between 18 and 80 g/m2, preferably between 20 and 75 g/m2, more preferably between 25 and 70 g/m2, even more preferably between 30 and 65 g/m2.

7. The absorbent article of any one of the previous claims, the top core wrap (110) comprises at least one spunbond and optionally at least one meltblown layer, wherein preferably the top core wrap consists of a spunbond layer.

8. The absorbent article of any one of the previous claims, wherein the top core wrap has a basis weight between 8 and 30 g/m2.

9. The absorbent article of any one of the previous claims, wherein the bottom core wrap (120, 800) is attached to the top core wrap (110) at least in a portion of the one or more channels, and optionally also to the capillary acceleration sheet at least in a portion of the one or more channels.

10. The absorbent article of the previous claim, wherein the bottom core wrap is attached to the top core wrap in one or more permanent attachment zones within the one or more channels; wherein, seen in a transverse direction of the absorbent core, an average width (wb) of each permanent attachment zone is smaller than an average width (wc) of the corresponding channel; and/or wherein, seen in a longitudinal direction of the absorbent core, an average length (lb) of each permanent attachment zone is smaller than an average length (lc) of the corresponding channel;
wherein preferably the average width (wb) of each permanent attachment zone is less than 90% of the average width (wc) of the corresponding channel, preferably less than 80%, more preferably less than 70%, and more than 25%.

11. The absorbent article of any one of the previous claims, wherein the bottom core wrap (120, 800) is in contact with the absorbent material and has an average density between 20 and 400 kg/m3, preferably between 20 and 300 kg/m3, more preferably between 20 and 250 kg/m3, even more preferably between 20 and 200 kg/m3, e.g. between 20 and 120 kg/m3; and/or wherein an amount of cellulosic fluff pulp in the absorbent material is below 250 g/m2, preferably below 200 g/m2, more preferably below 180 g/m2, even more preferably below 160 g/m2, e.g. between 60 g/m2 and 180 g/m2 or between 70 g/m2 and 170 g/m2; and/or wherein an amount of superabsorbent particles in the absorbent material is above 300 g/m2, preferably above 320 g/m2, more preferably above 340 g/m2, e.g. between 300 g/m2 and 400 g/m2 or between 320 g/m2 and 390 g/m2; and/or
wherein the superabsorbent particles have a permeability SFC which is higher than 10 x 10⁻⁷ cm³.S.g⁻¹ and/or a permeability GBP which is higher than 20 Da, preferably higher than 25 Da; and/or
wherein the absorbent material comprises more than 60 weight%, preferably more than 70 weight% superabsorbent particles.

12. The absorbent article of any one of the previous claims, wherein the bottom core wrap (120, 800) and/or the top core wrap (110) and/or the capillary acceleration sheet (400) comprises more than 50 weight% of synthetic fibres, preferably more than 80 weight% of synthetic fibres, preferably more than 90 weight% of synthetic fibres, even preferably more than 95weight% of synthetic fibres;
wherein preferably the synthetic fibres of the bottom core wrap comprise more than 20 weight% polyester fibres, preferably more than 30 weight% polyester fibres and/or more than 10 weight% polypropylene;
wherein preferably the bottom core wrap is any one of the following nonwovens: a resin bonded nonwoven, an air-through bonded nonwoven, an air-laid nonwoven, a needle bonded nonwoven, a spunbond through air-bond nonwoven, a 3D perforated film, a 3D embossed nonwoven, a hydro-entangled nonwoven, a thermo-carded nonwoven, or a combination thereof;
wherein preferably the bottom core wrap has a thickness between 0.1 and 2.5 mm, preferably between 0.2 and 1 mm, more preferably between 0.2 and 0.7 mm, e.g. between 0.2 and 0.5 mm;
wherein preferably the backsheet has a basis weight below 30 g/m2, preferably below 28 g/m2, e.g. below 26 g/m2 or even below 24 g/m2.

13. The absorbent article of any one of the previous claims, wherein the bottom core wrap (120, 800) comprises a first layer (801) and a second layer (802), wherein said first layer has a first density between 20 and 150 kg/m3 and said second layer has a second density between 100 and 400 kg/m3, said second density being higher than the first density, wherein said first layer is closer to the absorbent material than said second layer;
wherein preferably the second average density is selected such that it forms a barrier for the superabsorbent particles; and/or wherein the first density is selected such that superabsorbent particles are partially taken up in the first layer;
wherein preferably a difference between the first and the second density is higher than 20 kg/m3, preferably higher than 30 kg/m3 more preferably higher than 40 kg/m2, even more preferably between 50 and 150 kg/m3, most preferably between 60 and 140 kg/m3;
wherein preferably the first layer has a first basis weight and the second layer has a second basis weight, said second basis weight being lower than said first basis weight, wherein the difference between the first layer and the second layer is larger than 1 g/m2, preferably larger than 2 g/m2, more preferably larger than 5 g/m2;
wherein preferably the first layer has a first basis weight between 15 and 80 g/m2, preferably between 25 and 80 g/m2, and/or wherein the second layer has a second basis weight between 8 and 35 g/m2, preferably between 8 and 25 g/m2.

14. The absorbent article of claim 13, wherein the first layer comprises polyester fibres; and/or wherein the second layer comprises any one of the following fibres or a combination thereof: polyethylene, polyester, copolyester, polypropylene, polyactic acid (PLA).

15. The absorbent article of any one of the previous claims, wherein the one or more channels (140, 150, 160, 170, 180) comprise one or more elongated channels each having a channel length measured in a longitudinal direction of the absorbent core, said channel length being at least 10% of the length of the absorbent core, preferably at least 15% of the length of the absorbent core;
wherein preferably the one or more elongated channels comprise at least a first and a second elongated channel extending at a distance of each other in a substantially longitudinal direction of the absorbent core.

16. The absorbent article of any one of the previous claims, wherein the bottom core wrap (120, 800) is glued to the top core wrap (110) and/or a capillary acceleration sheet (400) at least in a portion of the one or more channels; and/or
wherein the bottom core wrap is sealed in accordance with a sealing pattern to the top core wrap and/or to the capillary acceleration sheet at least in a portion of the one or more channels, wherein preferably the sealing is realized by heat and/or pressure and/or ultrasonic energy; wherein preferably the sealing pattern covers less than 70%, preferably less than 60%, more preferably less than 50%, even more preferably between 1 and 40% of the surface area of the one or more channels; and/or wherein the sealing pattern is a regular pattern, such as a line pattern or a pattern of dots; and/or wherein the sealing pattern comprises a large number of distinct sealing areas, such as dots or lines, spread across the one or more channels, wherein the large number is larger than 10; and/or wherein the sealing pattern comprises a plurality of discrete elements, and wherein each element has a first dimension in a first direction and a second dimensions in a direction perpendicular to the first direction, and wherein the first dimension is smaller than 2 mm, preferably smaller than 1.5 mm, more preferably smaller than 1 mm, e.g. between 0.1 and 0.7 mm.

17. A method for manufacturing an absorbent article, comprising the steps of providing a liquid pervious topsheet (300) and a liquid impervious backsheet (200); arranging an absorbent material (130) comprising superabsorbent particles and optionally cellulosic fluff pulp, between a bottom core wrap (120, 800), on one side, and a top core wrap (110) and a capillary acceleration sheet (400), on the other side, such that one or more channels (140, 150, 160, 170, 180) are formed, wherein less absorbent material per surface area is present in the one or more channels compared to areas around the one or more channels, wherein preferably substantially no absorbent material is present in the one or more channels; and arranging the liquid pervious topsheet at the side of top core wrap and the capillary acceleration sheet and the liquid impervious backsheet at the side of the bottom core wrap, wherein the capillary acceleration (400) sheet has the features defined in any of the previous claims.

## Patentansprüche

1. Absorbierender Artikel, umfassend eine flüssigkeitsdurchlässige Oberlage (300), eine flüssigkeitsundurchlässige Unterlage (200), einen absorbierenden Kern (100), der zwischen der flüssigkeitsdurchlässigen Oberlage und der flüssigkeitsundurchlässigen Unterlage positioniert ist, der absorbierende Kern umfassend eine untere Kernumhüllung (120, 800), eine obere Kernumhüllung (110) und absorbierendes Material (130) zwischen der unteren Kernumhüllung und der oberen Kernumhüllung und eine Kapillarbeschleunigungslage (400) zwischen dem absorbierenden Kern und der flüssigkeitsdurchlässigen Oberlage (300);
wobei das absorbierende Material (130) superabsorbierende Partikel und optional Zellulose-Flockenzellstoff umfasst, wobei das absorbierende Material derart angeordnet ist, dass ein oder mehrere Kanäle (140, 150, 160, 170, 180) ausgebildet werden, wobei in dem einen oder den mehreren Kanälen weniger absorbierendes Material pro Oberflächenbereich im Vergleich zu einem Bereich um den einen oder die mehreren Kanäle herum vorhanden ist, wobei vorzugsweise in dem einen oder den mehreren Kanälen im Wesentlichen kein absorbierendes Material vorhanden ist, wobei die Kapillarbeschleunigungslage (400) ein durchluftgebundenes Vlies ist, das ein Flächengewicht zwischen 20 und 30 gsm aufweist.

2. Absorbierender Artikel nach Anspruch 1, wobei die Kapillarbeschleunigungslage (400) einer hydrophilen Spinnfinish-Behandlung unterzogen wurde.

3. Absorbierender Artikel nach einem der vorstehenden Ansprüche, wobei der eine oder die mehreren Kanäle ein oder mehrere längliche Kanäle sind, die eine Länge aufweisen, die in Längsrichtung des absorbierenden Kerns gemessen wird, und wobei jeder Kanal eine Breite aufweist, die in Querrichtung des absorbierenden Kerns gemessen wird, wobei die Breite mindestens 5 % der Breite des absorbierenden Kerns beträgt, vorzugsweise mindestens 7 %, z. B. mindestens 10 %, und weniger als 30 %.

4. Absorbierender Artikel nach einem der vorstehenden Ansprüche, wobei die untere Kernumhüllung (120, 800) aus einer einzigen Lage besteht.

5. Absorbierender Artikel nach einem der vorstehenden Ansprüche, wobei die untere Kernumhüllung (120, 800) ein durchluftgebundenes Vlies umfasst, wobei vorzugsweise die untere Kernumhüllung aus einem durchluftgebundenen Vlies besteht.

6. Absorbierender Artikel nach einem der vorstehenden Ansprüche, wobei die untere Kernumhüllung (120, 800) ein Flächengewicht zwischen 18 und 80 g/m2, vorzugsweise zwischen 20 und 75 g/m2, mehr bevorzugt zwischen 25 und 70 g/m2, noch mehr bevorzugt zwischen 30 und 65 g/m2, aufweist.

7. Absorbierender Artikel nach einem der vorstehenden Ansprüche, wobei die obere Kernumhüllung (110) mindestens eine Spinnvlies- und optional mindestens eine Schmelzblasschicht umfasst, wobei vorzugsweise die obere Kernumhüllung aus einer Spinnvliesschicht besteht.

8. Absorbierender Artikel nach einem der vorstehenden Ansprüche, wobei die obere Kernumhüllung ein Flächengewicht zwischen 8 und 30 g/m2 aufweist.

9. Absorbierender Artikel nach einem der vorstehenden Ansprüche, wobei die untere Kernumhüllung (120, 800) mindestens in einem Abschnitt des einen oder der mehreren Kanäle an der oberen Kernumhüllung (110) und optional ebenso mindestens in einem Abschnitt des einen oder der mehreren Kanäle an der Kapillarbeschleunigungslage befestigt ist.

10. Absorbierender Artikel des vorstehenden Anspruchs, wobei die untere Kernumhüllung in einer oder mehreren permanenten Befestigungszonen innerhalb des einen oder der mehreren Kanäle an der oberen Kernumhüllung befestigt ist; wobei, in einer Querrichtung des absorbierenden Kerns gesehen, eine durchschnittliche Breite (wb) jeder permanenten Befestigungszone kleiner als eine durchschnittliche Breite (wc) des entsprechenden Kanals ist; und/oder wobei, in einer Längsrichtung des absorbierenden Kerns gesehen, eine durchschnittliche Länge (1b) jeder permanenten Befestigungszone kleiner als eine durchschnittliche Länge (1c) des entsprechenden Kanals ist;
wobei vorzugsweise die durchschnittliche Breite (wb) jeder permanenten Befestigungszone weniger als 90 % der durchschnittlichen Breite (wc) des entsprechenden Kanals, vorzugsweise weniger als 80 %, mehr bevorzugt weniger als 70 % und mehr als 25 %, beträgt.

11. Absorbierender Artikel nach einem der vorstehenden Ansprüche, wobei die untere Kernumhüllung (120, 800) mit dem absorbierenden Material in Kontakt steht und eine durchschnittliche Dichte zwischen 20 und 400 kg/m3, vorzugsweise zwischen 20 und 300 kg/m3, mehr bevorzugt zwischen 20 und 250 kg/m3, noch mehr bevorzugt zwischen 20 und 200 kg/m3, z. B. zwischen 20 und 120 kg/m3, aufweist; und/oder wobei eine Menge an Zellulose-Flockenzellstoff in dem absorbierenden Material unter 250 g/m2, vorzugsweise unter 200 g/m2, mehr bevorzugt unter 180 g/m2, noch mehr bevorzugt unter 160 g/m2, z. B. zwischen 60 g/m2 und 180 g/m2 oder zwischen 70 g/m2 und 170 g/m2, liegt; und/oder wobei eine Menge an superabsorbierenden Partikeln in dem absorbierenden Material über 300 g/m2, vorzugsweise über 320 g/m2, mehr bevorzugt über 340 g/m2, z. B. zwischen 300 g/m2 und 400 g/m2 oder zwischen 320 g/m2 und 390 g/m2, liegt; und/oder
wobei die superabsorbierenden Partikel eine Permeabilitäts-SFC, die höher als 10 x 10⁻⁷ cm³.Sg⁻¹ ist und/oder ein Permeabilitäts-GBP aufweisen, die höher als 20 Da, vorzugsweise höher als 25 Da ist; und/oder
wobei das absorbierende Material zu mehr als 60 Gew.-%, vorzugsweise zu mehr als 70 Gew.-% superabsorbierende Partikel umfasst.

12. Absorbierender Artikel nach einem der vorsehenden Ansprüche, wobei die untere Kernumhüllung (120, 800) und/oder die obere Kernumhüllung (110) und/oder die Kapillarbeschleunigungslage (400) mehr als zu 50 Gew.-% synthetische Fasern, vorzugsweise mehr als zu 80 Gew.-% synthetische Fasern, vorzugsweise mehr als zu 90 Gew.-% synthetische Fasern, noch mehr bevorzugt mehr als zu 95 Gew.-% synthetische Fasern umfasst;
wobei vorzugsweise die synthetischen Fasern der unteren Kernummantelung mehr als zu 20 Gew.-% Polyesterfasern, vorzugsweise mehr als zu 30 Gew.-% Polyesterfasern und/oder mehr als zu 10 Gew.-% Polypropylen umfassen;
wobei vorzugsweise die untere Kernumhüllung ein beliebiges der folgenden Vliese ist: ein harzgebundenes Vlies, ein durchluftgebundenes Vlies, ein luftgelegtes Vlies, ein nadelgebundenes Vlies, ein durchluftgebundenes Spinnvlies, eine 3D-perforierte Folie, ein 3D-geprägtes Vlies, ein hydroverfestigtes Vlies, ein thermokardiertes Vlies oder eine Kombination davon;
wobei vorzugsweise die untere Kernumhüllung eine Dicke zwischen 0,1 und 2,5 mm, vorzugsweise zwischen 0,2 und 1 mm, mehr bevorzugt zwischen 0,2 und 0,7 mm, z. B. zwischen 0,2 und 0,5 mm, aufweist;
wobei vorzugsweise die Unterlage ein Flächengewicht unter 30 g/m2, vorzugsweise unter 28 g/m2, z. B. unter 26 g/m2 oder sogar unter 24 g/m2 aufweist.

13. Absorbierender Artikel nach einem der vorstehenden Ansprüche, wobei die untere Kernumhüllung (120, 800) eine erste Schicht (801) und eine zweite Schicht (802) umfasst, wobei die erste Schicht eine erste Dichte zwischen 20 und 150 kg/m3 aufweist und die zweite Schicht eine zweite Dichte zwischen 100 und 400 kg/m3 aufweist, wobei die zweite Dichte höher als die erste Dichte ist, wobei die erste Schicht näher an dem absorbierenden Material als die zweite Schicht liegt;
wobei vorzugsweise die zweite Durchschnittsdichte derart ausgewählt ist, dass sie eine Barriere für die superabsorbierenden Partikel ausbildet; und/oder wobei die erste Dichte derart ausgewählt ist, dass superabsorbierende Partikel teilweise in der ersten Schicht aufgenommen werden;
wobei vorzugsweise ein Unterschied zwischen der ersten und der zweiten Dichte größer als 20 kg/m3, vorzugsweise größer als 30 kg/m3, mehr bevorzugt größer als 40 kg/m2, noch mehr bevorzugt zwischen 50 und 150 kg/m3, am meisten bevorzugt zwischen 60 und 140 kg/m3, ist;
wobei vorzugsweise die erste Schicht ein erstes Flächengewicht und die zweite Schicht ein zweites Flächengewicht aufweist, wobei das zweite Flächengewicht geringer als das erste Flächengewicht ist, wobei der Unterschied zwischen der ersten Schicht und der zweiten Schicht größer als 1 g/m2, vorzugsweise größer als 2 g/m2, mehr bevorzugt größer als 5 g/m2, ist;
wobei vorzugsweise die erste Schicht ein erstes Flächengewicht zwischen 15 und 80 g/m2, vorzugsweise zwischen 25 und 80 g/m2, aufweist und/oder wobei die zweite Schicht ein zweites Flächengewicht zwischen 8 und 35 g/m2, vorzugsweise zwischen 8 und 25 g/m2, aufweist.

14. Absorbierender Artikel nach Anspruch 13, wobei die erste Schicht Polyesterfasern umfasst; und/oder wobei die zweite Schicht eine beliebige der folgenden Fasern oder eine Kombination davon umfasst: Polyethylen, Polyester, Copolyester, Polypropylen, Polymilchsäure (PLA).

15. Absorbierender Artikel nach einem der vorstehenden Ansprüche, wobei der eine oder die mehreren Kanäle (140, 150, 160, 170, 180) einen oder mehrere längliche Kanäle umfassen, die jeweils eine Kanallänge aufweisen, die in einer Längsrichtung des absorbierenden Kerns gemessen wird, wobei die Kanallänge mindestens 10 % der Länge des absorbierenden Kerns, vorzugsweise mindestens 15 % der Länge des absorbierenden Kerns, beträgt;
wobei vorzugsweise der eine oder die mehreren länglichen Kanäle mindestens einen ersten und einen zweiten länglichen Kanal umfassen, die sich in einem Abstand voneinander in einer im Wwesentlichen Längsrichtung des absorbierenden Kerns erstrecken.

16. Absorbierender Artikel nach einem der vorstehenden Ansprüche, wobei die untere Kernumhüllung (120, 800) mit der oberen Kernumhüllung (110) verklebt ist und/oder eine Kapillarbeschleunigungslage (400) mindestens in einem Abschnitt des einen oder der mehreren Kanäle vorliegt; und/oder
wobei die untere Kernumhüllung gemäß einem Versiegelungsmuster mit der oberen Kernumhüllung und/oder mit der Kapillarbeschleunigungslage mindestens in einem Abschnitt des einen oder der mehreren Kanäle versiegelt ist, wobei vorzugsweise die Versiegelung durch Wärme und/oder Druck und/oder Ultraschallenergie erfolgt;
wobei vorzugsweise das Versiegelungsmuster weniger als 70 %, vorzugsweise weniger als 60 %, mehr bevorzugt weniger als 50 %, noch mehr bevorzugt zwischen 1 und 40 % des Oberflächenbereichs des einen oder der mehreren Kanäle bedeckt; und/oder wobei das Versiegelungsmuster ein regelmäßiges Muster ist, wie ein Linienmuster oder ein Muster von Punkten; und/oder wobei das Versiegelungsmuster eine große Anzahl unterschiedlicher Versiegelungsbereiche, wie Punkte oder Linien, umfasst, die über den einen oder die mehreren Kanäle verteilt sind, wobei die große Anzahl größer als 10 ist; und/oder wobei das Versiegelungsmuster eine Vielzahl von diskreten Elementen umfasst und wobei jedes Element eine erste Abmessung in einer ersten Richtung und eine zweite Abmessungen in einer Richtung senkrecht zu der ersten Richtung aufweist und wobei die erste Abmessung kleiner als 2 mm, vorzugsweise kleiner als 1,5 mm, mehr bevorzugt kleiner als 1 mm, z. B. zwischen 0,1 und 0,7 mm, ist.

17. Verfahren zum Herstellen eines absorbierenden Artikels, umfassend die Schritte eines Bereitstellens einer flüssigkeitsdurchlässigen Oberlage (300) und einer flüssigkeitsundurchlässigen Unterlage (200); Anordnen eines absorbierenden Materials (130), umfassend superabsorbierende Partikel und optional Zellulose-Flockenzellstoff, zwischen einer unteren Kernumhüllung (120, 800), auf einer Seite, und einer oberen Kernumhüllung (110) und einer Kapillarbeschleunigungslage (400), auf der anderen Seite, derart, dass ein oder mehrere Kanäle (140, 150, 160, 170, 180) ausgebildet werden, wobei in dem einen oder den mehreren Kanälen weniger absorbierendes Material pro Oberflächenbereich im Vergleich zu den Bereichen um den einen oder die mehreren Kanäle herum vorhanden ist, wobei vorzugsweise in dem einen oder den mehreren Kanälen im Wesentlichen kein absorbierendes Material vorhanden ist; und Anordnen der flüssigkeitsdurchlässigen Oberlage an der Seite der oberen Kernumhüllung und der Kapillarbeschleunigungslage und der flüssigkeitsundurchlässigen Unterlage an der Seite der unteren Kernumhüllung, wobei die Kapillarbeschleunigungs(400)-Lage die Merkmale aufweist, die in einem der vorstehenden Ansprüche definiert sind.

## Revendications

1. Article absorbant comprenant une feuille supérieure (300) perméable aux liquides, une feuille arrière (200) imperméable aux liquides, un noyau absorbant (100) positionné entre la feuille supérieure perméable aux liquides et la feuille arrière imperméable aux liquides, ledit noyau absorbant comprenant une enveloppe centrale inférieure (120, 800), une enveloppe centrale supérieure (110) et un matériau absorbant (130) entre l'enveloppe centrale inférieure et l'enveloppe centrale supérieure, et une feuille d'accélération capillaire (400) entre le noyau absorbant et la feuille supérieure (300) perméable aux liquides ;
dans lequel le matériau absorbant (130) comprend des particules superabsorbantes et éventuellement de la pâte cellulosique défibrée, dans lequel le matériau absorbant est disposé de telle sorte qu'un ou plusieurs canaux (140, 150, 160, 170, 180) sont formés, dans lequel moins de matériau absorbant par aire surfacique est présent dans le ou les canaux par rapport à une aire autour du ou des canaux, dans lequel de préférence pratiquement aucun matériau absorbant n'est présent dans le ou les canaux, dans lequel la feuille d'accélération capillaire (400) étant un non-tissé collé à l'air présentant un poids de base entre 20 et 30 g/m².

2. Article absorbant selon la revendication 1, dans lequel la feuille d'accélération capillaire (400) a été soumise à un traitement d'ensimage de filature hydrophile.

3. Article absorbant selon l'une quelconque des revendications précédentes, dans lequel le ou les canaux sont un ou plusieurs canaux allongés présentant une longueur mesurée dans une direction longitudinale du noyau absorbant et dans lequel chaque canal présente une largeur mesurée dans une direction transversale du noyau absorbant, ladite largeur représentant au moins 5 % de la largeur du noyau absorbant, de préférence au moins 7 %, par exemple au moins 10 %, et moins de 30 %.

4. Article absorbant selon l'une quelconque des revendications précédentes, dans lequel l'enveloppe centrale inférieure (120, 800) est constituée d'une seule feuille.

5. Article absorbant selon l'une quelconque des revendications précédentes, dans lequel l'enveloppe centrale inférieure (120, 800) comprend un non-tissé collé à l'air, dans lequel de préférence l'enveloppe centrale inférieure est constituée d'un non-tissé collé à l'air.

6. Article absorbant selon l'une quelconque des revendications précédentes, dans lequel l'enveloppe centrale inférieure (120, 800) présente un poids de base entre 18 et 80 g/m², de préférence entre 20 et 75 g/m², plus préférablement entre 25 et 70 g/m², encore plus préférablement entre 30 et 65 g/m².

7. Article absorbant selon l'une quelconque des revendications précédentes, l'enveloppe centrale supérieure (110) comprend au moins une couche de filé-lié et éventuellement au moins une couche obtenue par fusion-soufflage, dans lequel de préférence l'enveloppe centrale supérieure est constituée d'une couche de filé-lié.

8. Article absorbant selon l'une quelconque des revendications précédentes, dans lequel l'enveloppe centrale supérieure présente un poids de base entre 8 et 30 g/m².

9. Article absorbant selon l'une quelconque des revendications précédentes, dans lequel l'enveloppe centrale inférieure (120, 800) est fixée à l'enveloppe centrale supérieure (110) au moins dans une partie du ou des canaux, et éventuellement aussi à la feuille d'accélération capillaire au moins dans une partie du ou des canaux.

10. Article absorbant selon la revendication précédente, dans lequel l'enveloppe centrale inférieure est fixée à l'enveloppe centrale supérieure dans une ou plusieurs zones de fixation permanente à l'intérieur du ou des canaux ; dans lequel, vu dans une direction transversale du noyau absorbant, une largeur moyenne (wb) de chaque zone de fixation permanente est inférieure à une largeur moyenne (wc) du canal correspondant ; et/ou dans lequel, vu dans une direction longitudinale du noyau absorbant, une longueur moyenne (1b) de chaque zone de fixation permanente est inférieure à une longueur moyenne (1c) du canal correspondant ;
dans lequel de préférence la largeur moyenne (wb) de chaque zone de fixation permanente est inférieure à 90 % de la largeur moyenne (wc) du canal correspondant, de préférence inférieure à 80 %, plus préférablement inférieure à 70 %, et supérieure à 25 %.

11. Article absorbant selon l'une quelconque des revendications précédentes, dans lequel l'enveloppe centrale inférieure (120, 800) est en contact avec le matériau absorbant et présente une masse volumique moyenne entre 20 et 400 kg/m³, de préférence entre 20 et 300 kg/m³, plus préférablement entre 20 et 250 kg/m³, encore plus préférablement entre 20 et 200 kg/m³, par exemple entre 20 et 120 kg/m³ ; et/ou dans lequel une quantité de pâte cellulosique défibrée dans le matériau absorbant est inférieure à 250 g/m², de préférence inférieure à 200 g/m², plus préférablement inférieure à 180 g/m², encore plus préférablement inférieure à 160 g/m², par exemple entre 60 g/m² et 180 g/m² ou entre 70 g/m² et 170 g/m² ; et/ou dans lequel une quantité de particules superabsorbantes dans le matériau absorbant est supérieure à 300 g/m², de préférence supérieure à 320 g/m², plus préférablement supérieure à 340 g/m², par exemple entre 300 g/m² et 400 g/m² ou entre 320 g/m² et 390 g/m² ; et/ou
dans lequel les particules superabsorbantes présentent une perméabilité SFC (chromatographie en phase supercritique) qui est supérieure à 10 x 10⁻⁷ cm³.S.g⁻¹ et/ou une perméabilité GBP (perméabilité à travers un lit de gel) qui est supérieure à 20 Da, de préférence supérieure à 25 Da ; et/ou
dans lequel le matériau absorbant comprend plus de 60 % en poids, de préférence plus de 70 % en poids de particules superabsorbantes.

12. Article absorbant selon l'une quelconque des revendications précédentes, dans lequel l'enveloppe centrale inférieure (120, 800) et/ou l'enveloppe centrale supérieure (110) et/ou la feuille d'accélération capillaire (400) comprend plus de 50 % en poids de fibres synthétiques, de préférence plus de 80 % en poids de fibres synthétiques, de préférence plus de 90 % en poids de fibres synthétiques, de préférence encore plus de 95 % en poids de fibres synthétiques ;
dans lequel de préférence les fibres synthétiques de l'enveloppe centrale inférieure comprennent plus de 20 % en poids de fibres de polyester, de préférence plus de 30 % en poids de fibres de polyester et/ou plus de 10 % en poids de polypropylène ;
dans lequel de préférence l'enveloppe centrale inférieure est un quelconque non-tissé parmi les non-tissés suivants : un non-tissé lié par une résine, un non-tissé collé à l'air, un non-tissé formé par voie sèche, un non-tissé aiguilleté, un non-tissé collé à l'air filé-lié, un film perforé en 3D, un non-tissé gaufré en 3D, un non-tissé hydro-enchevêtré, un non-tissé thermo-cardé, ou une combinaison de ceux-ci ;
dans lequel de préférence l'enveloppe centrale inférieure présente une épaisseur entre 0,1 et 2,5 mm, de préférence entre 0,2 et 1 mm, plus préférablement entre 0,2 et 0,7 mm, par exemple entre 0,2 et 0,5 mm ;
dans lequel de préférence la feuille arrière présente un poids de base inférieur à 30 g/m², de préférence inférieur à 28 g/m², par exemple inférieur à 26 g/m² ou même inférieur à 24 g/m².

13. Article absorbant selon l'une quelconque des revendications précédentes, dans lequel l'enveloppe centrale inférieure (120, 800) comprend une première couche (801) et une seconde couche (802), dans lequel ladite première couche présente une première masse volumique entre 20 et 150 kg/m³ et ladite seconde couche présente une seconde masse volumique entre 100 et 400 kg/m³, ladite seconde masse volumique étant supérieure à la première masse volumique, dans lequel ladite première couche est plus proche du matériau absorbant que ladite seconde couche ;
dans lequel de préférence la seconde masse volumique moyenne est choisie de telle sorte qu'elle forme une barrière aux particules superabsorbantes ; et/ou dans lequel la première masse volumique est choisie de telle sorte que des particules superabsorbantes sont partiellement absorbées dans la première couche ;
dans lequel de préférence une différence entre la première et la seconde masse volumique est supérieure à 20 kg/m³, de préférence supérieure à 30 kg/m³, plus préférablement supérieure à 40 kg/m², encore plus préférablement entre 50 et 150 kg/m³, le plus préférablement entre 60 et 140 kg/m³ ;
dans lequel de préférence la première couche présente un premier poids de base et la seconde couche présente un second poids de base, ledit second poids de base étant inférieur audit premier poids de base, dans lequel la différence entre la première couche et la seconde couche est supérieure à 1 g/m², de préférence supérieure à 2 g/m², plus préférablement supérieure à 5 g/m² ;
dans lequel de préférence la première couche présente un premier poids de base entre 15 et 80 g/m², de préférence entre 25 et 80 g/m² et/ou dans lequel la seconde couche présente un second poids de base entre 8 et 35 g/m², de préférence entre 8 et 25 g/m².

14. Article absorbant selon la revendication 13, dans lequel la première couche comprend des fibres de polyester ; et/ou dans lequel la seconde couche comprend une quelconque fibre parmi les fibres suivantes ou une combinaison de celles-ci : polyéthylène, polyester, copolyester, polypropylène, acide polylactique (PLA).

15. Article absorbant selon l'une quelconque des revendications précédentes, dans lequel le ou les canaux (140, 150, 160, 170, 180) comprennent un ou plusieurs canaux allongés présentant chacun une longueur de canal mesurée dans une direction longitudinale du noyau absorbant, ladite longueur de canal représentant au moins 10 % de la longueur du noyau absorbant, de préférence au moins 15 % de la longueur du noyau absorbant ;
dans lequel de préférence le ou les canaux allongés comprennent au moins un premier et un second canal allongé s'étendant à une certaine distance l'un de l'autre dans une direction essentiellement longitudinale du noyau absorbant.

16. Article absorbant selon l'une quelconque des revendications précédentes, dans lequel l'enveloppe centrale inférieure (120, 800) est collée à l'enveloppe centrale supérieure (110) et/ou à une feuille d'accélération capillaire (400) au moins dans une partie du ou des canaux ; et/ou
dans lequel l'enveloppe centrale inférieure est scellée selon un modèle de scellage contre l'enveloppe centrale supérieure et/ou la feuille d'accélération capillaire au moins dans une partie du ou des canaux, dans lequel de préférence le scellage est réalisé par la chaleur et/ou la pression et/ou l'énergie ultrasonique ;
dans lequel de préférence le motif de scellage couvre moins de 70 %, de préférence moins de 60 %, plus préférablement moins de 50 %, encore plus préférablement entre 1 et 40 % de l'aire surfacique du ou des canaux ; et/ou dans lequel le motif de scellage est un motif régulier, tel qu'un motif de lignes ou un motif de points ; et/ou dans lequel le motif de scellage comprend un grand nombre d'aires de scellage distinctes, telles que des points ou des lignes, réparties sur le ou les canaux, dans lequel le grand nombre est supérieur à 10 ; et/ou dans lequel le motif de scellage comprend une pluralité d'éléments discrets et dans lequel chaque élément présente une première dimension dans une première direction et une seconde dimension dans une direction perpendiculaire à la première direction, et dans lequel la première dimension est inférieure à 2 mm, de préférence inférieure à 1,5 mm, plus préférablement inférieure à 1 mm, par exemple entre 0,1 et 0,7 mm.

17. Procédé de fabrication d'un article absorbant, comprenant les étapes consistant à fournir une feuille supérieure (300) perméable aux liquides et une feuille arrière (200) imperméable aux liquides ; disposer un matériau absorbant (130) comprenant des particules superabsorbantes et éventuellement de la pâte cellulosique défibrée, entre une enveloppe centrale inférieure (120, 800), d'un côté, et une enveloppe centrale supérieure (110) et une feuille d'accélération capillaire (400), de l'autre côté, de telle sorte qu'un ou plusieurs canaux (140, 150, 160, 170, 180) sont formés, dans lequel moins de matériau absorbant par aire surfacique est présent dans le ou les canaux par rapport aux aires autour du ou des canaux, dans lequel de préférence essentiellement aucun matériau absorbant n'est présent dans le ou les canaux ; et disposer la feuille supérieure perméable aux liquides sur le côté de l'enveloppe centrale supérieure et la feuille d'accélération capillaire et la feuille arrière imperméable aux liquides sur le côté de l'enveloppe centrale inférieure, la feuille d'accélération capillaire (400) présentant les caractéristiques définies dans l'une quelconque des revendications précédentes.
